# EUROPEAN PATENT APPLICATION

(11) **EP 3 616 717 A1**
(43) Date of publication of application: **04.03.2020**
(21) Application number: 18191922.6
(22) Date of filing: 31.08.2018
(51) Int. Cl.: A61K 39/10, A61P 31/04, C07K 14/235

(54) **RECOMBINANT ANTIGEN INDUCING ANTIBODIES FOR NEUTRALIZATION OF BORDETELLA ADENYLATE CYCLASE TOXIN**

(71) Applicant: Mikrobiologicky ustav AV CR, v.v.i., 142 20 Praha 4 - Krc (CZ)
(72) Inventor: Sebo, Peter, 14200 Praha 4 - Krc (CZ); Bumba, Ladislav, 14200 Praha 4 - Krc (CZ); Espinosa Vinals, Carlos Angel, 14200 Praha 4 - Krc (CZ); Holubova, Jana, 14220 Praha 4 - Krc (CZ); Stanek, Ondrej, 14200 Praha 4 - Krc (CZ); Osicka, Radim, 14200 Praha 4 - Krc (CZ); Masin, Jiri, 14220 Praha 4 - Krc (CZ)
(74) Representative: Hartvichova, Katerina

(57) **Abstract**

The present invention provides a polypeptide antigen comprising amino acid sequences derived from adenylate cyclase protein of *Bordetella pertussis,* comprising (i) an amino acid sequence of a fragment of CyaA, having on its N-terminus an amino acid residue in position between 1132-1166 and having on its C-terminus an amino acid residue in position between 1287-1294, and to its C-terminus fused: (ii) an amino acid sequence of a fragment of CyaA, having on its N-terminus an amino acid residue in position between 1561-1570 and having on its C-terminus an amino acid residue in position between 1680-1681. N-terminally extended derivatives of this sequence are also provided.

Said polypeptide antigen is suitable for use in a method of treatment and/or prevention of a disease caused by *Bordetella sp.*, in particular *Bordetella pertussis, Bordetella parapertussis*, or *Bordetella bronchiseptica*, and in particular against pertussis or whooping cough disease. The invention further provides a chimeric protein, a polynucleotide encoding the polypeptide antigen or chimeric protein, a vector and a host cell for the production of said polypeptide or protein.

SEQUENCE LISTING

<110>  Mikrobiologicky ustav AV CR, v.v.i.

<120>  Recombinant antigen inducing antibodies for neutralization of
       Bordetella adenylate cyclase toxin

<130>  P

<160>  13

<170>  PatentIn version 3.5

<210>  1
<211>  1706
<212>  PRT
<213>  Bordetella pertussis

<400>  1

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala

675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                 1005

His Val  Gln His Ile Ile Gly  Gly Ala Gly Asn Asp  Ser Ile Thr
    1010                 1015                 1020

Gly Asn  Ala His Asp Asn Phe  Leu Ala Gly Gly Ser  Gly Asp Asp
    1025                 1030                 1035

Arg Leu  Asp Gly Gly Ala Gly  Asn Asp Thr Leu Val  Gly Gly Glu
    1040                 1045                 1050

Gly Gln  Asn Thr Val Ile Gly  Gly Ala Gly Asp Asp  Val Phe Leu
    1055                 1060                 1065

Gln Asp  Leu Gly Val Trp Ser  Asn Gln Leu Asp Gly  Gly Ala Gly
    1070                 1075                 1080

Val Asp  Thr Val Lys Tyr Asn  Val His Gln Pro Ser  Glu Glu Arg
    1085                 1090                 1095

Leu Glu  Arg Met Gly Asp Thr  Gly Ile His Ala Asp  Leu Gln Lys
    1100                 1105                 1110

Gly Thr  Val Glu Lys Trp Pro  Ala Leu Asn Leu Phe  Ser Val Asp
    1115                 1120                 1125

His Val  Lys Asn Ile Glu Asn  Leu His Gly Ser Arg  Leu Asn Asp
    1130                 1135                 1140

Arg Ile  Ala Gly Asp Asp Gln  Asp Asn Glu Leu Trp  Gly His Asp
    1145                 1150                 1155

Gly Asn  Asp Thr Ile Arg Gly  Arg Gly Gly Asp Asp  Ile Leu Arg
    1160                 1165                 1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Val Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val

1640                1645                1650

Glu Ile  Ile His Ala Ala Asn  Gln Ala Val Asp Gln  Ala Gly Ile
    1655                1660                1665

Glu Lys  Leu Val Glu Ala Met  Ala Gln Tyr Pro Asp  Pro Gly Ala
    1670                1675                1680

Ala Ala  Ala Ala Pro Pro Ala  Ala Arg Val Pro Asp  Thr Leu Met
    1685                1690                1695

Gln Ser  Leu Ala Val Asn Trp  Arg
    1700                1705

<210>  2
<211>  283
<212>  PRT
<213>  artificial

<220>
<223>  polypeptide antigen RTXm

<400>  2

Asn Ile Glu Asn Leu His Gly Ser Arg Leu Asn Asp Arg Ile Ala Gly
1               5                   10                  15

Asp Asp Gln Asp Asn Glu Leu Trp Gly His Asp Gly Asn Asp Thr Ile
            20                  25                  30

Arg Gly Arg Gly Gly Asp Asp Ile Leu Arg Gly Gly Leu Gly Leu Asp
        35                  40                  45

Thr Leu Tyr Gly Glu Asp Gly Asn Asp Ile Phe Leu Gln Asp Asp Glu
    50                  55                  60

Thr Val Ser Asp Asp Ile Asp Gly Gly Ala Gly Leu Asp Thr Val Asp
65                  70                  75                  80

Tyr Ser Ala Met Ile His Pro Gly Arg Ile Val Ala Pro His Glu Tyr
                85                  90                  95

Gly Phe Gly Ile Glu Ala Asp Leu Ser Arg Glu Trp Val Arg Lys Ala
            100                 105                 110

Ser Ala Leu Gly Val Asp Tyr Tyr Asp Asn Val Arg Asn Val Glu Asn
        115                 120                 125

Val Ile Gly Thr Ser Met Lys Asp Val Leu Ile Gly Asp Ala Gln Ala
    130                 135                 140

Asn Thr Leu Met Gly Gln Gly Gly Asp Asp Thr Val Arg Gly Gly Asp
145                 150                 155                 160

Gly Asp Asp Val Leu Leu Gly Asp Glu Gly Ser Asp Leu Leu Ser Gly
                165                 170                 175

Asp Ala Gly Asn Asp Asp Leu Phe Gly Gly Gln Gly Asp Asp Thr Tyr
            180                 185                 190

Leu Phe Gly Val Gly Tyr Gly His Asp Thr Ile Tyr Glu Ser Gly Gly
        195                 200                 205

Gly His Asp Thr Ile Arg Ile Asn Ala Gly Ala Asp Gln Leu Trp Phe
    210                 215                 220

Ala Arg Gln Gly Asn Asp Leu Glu Ile Arg Ile Leu Gly Thr Asp Asp
225                 230                 235                 240

Ala Leu Thr Val His Asp Trp Tyr Arg Asp Ala Asp His Arg Val Glu
                245                 250                 255

Ile Ile His Ala Ala Asn Gln Ala Val Asp Gln Ala Gly Ile Glu Lys
            260                 265                 270

Leu Val Glu Ala Met Ala Gln Tyr Pro Asp Pro
        275                 280

<210>  3
<211>  432
<212>  PRT
<213>  artificial

<220>
<223>  RTX1008 polypeptide antigen

<400>  3

Glu His Val Gln His Ile Ile Gly Gly Ala Gly Asn Asp Ser Ile Thr
1               5                   10                  15

Gly Asn Ala His Asp Asn Phe Leu Ala Gly Gly Ser Gly Asp Asp Arg
            20                  25                  30

Leu Asp Gly Gly Ala Gly Asn Asp Thr Leu Val Gly Gly Glu Gly Gln
        35                  40                  45

Asn Thr Val Ile Gly Gly Ala Gly Asp Asp Val Phe Leu Gln Asp Leu
    50                  55                  60

Gly Val Trp Ser Asn Gln Leu Asp Gly Gly Ala Gly Val Asp Thr Val
65                  70                  75                  80

Lys Tyr Asn Val His Gln Pro Ser Glu Glu Arg Leu Glu Arg Met Gly
                85                  90                  95

Asp Thr Gly Ile His Ala Asp Leu Gln Lys Gly Thr Val Glu Lys Trp
            100                 105                 110

Pro Ala Leu Asn Leu Phe Ser Val Asp His Val Lys Asn Ile Glu Asn
        115                 120                 125

Leu His Gly Ser Arg Leu Asn Asp Arg Ile Ala Gly Asp Asp Gln Asp
    130                 135                 140

Asn Glu Leu Trp Gly His Asp Gly Asn Asp Thr Ile Arg Gly Arg Gly
145                 150                 155                 160

Gly Asp Asp Ile Leu Arg Gly Gly Leu Gly Leu Asp Thr Leu Tyr Gly
                165                 170                 175

Glu Asp Gly Asn Asp Ile Phe Leu Gln Asp Asp Glu Thr Val Ser Asp
            180                 185                 190

Asp Ile Asp Gly Gly Ala Gly Leu Asp Thr Val Asp Tyr Ser Ala Met
        195                 200                 205

Ile His Pro Gly Arg Ile Val Ala Pro His Glu Tyr Gly Phe Gly Ile
    210                 215                 220

Glu Ala Asp Leu Ser Arg Glu Trp Val Arg Lys Ala Ser Ala Leu Gly
225                 230                 235                 240

Val Asp Tyr Tyr Asp Asn Val Arg Asn Val Glu Asn Val Ile Gly Thr
                245                 250                 255

Ser Met Lys Asp Val Leu Ile Gly Asp Ala Gln Ala Asn Thr Leu Met
            260                 265                 270

Gly Gln Gly Gly Asp Asp Thr Val Arg Gly Gly Asp Gly Asp Asp Val
        275                 280                 285

Leu Leu Gly Asp Glu Gly Ser Asp Leu Leu Ser Gly Asp Ala Gly Asn
    290                 295                 300

Asp Asp Leu Phe Gly Gly Gln Gly Asp Asp Thr Tyr Leu Phe Gly Val
305                 310                 315                 320

Gly Tyr Gly His Asp Thr Ile Tyr Glu Ser Gly Gly Gly His Asp Thr
                325                 330                 335

Ile Arg Ile Asn Ala Gly Ala Asp Gln Leu Trp Phe Ala Arg Gln Gly
            340                 345                 350

Asn Asp Leu Glu Ile Arg Ile Leu Gly Thr Asp Asp Ala Leu Thr Val
        355                 360                 365

His Asp Trp Tyr Arg Asp Ala Asp His Arg Val Glu Ile Ile His Ala
    370                 375                 380

Ala Asn Gln Ala Val Asp Gln Ala Gly Ile Glu Lys Leu Val Glu Ala
385                 390                 395                 400

Met Ala Gln Tyr Pro Asp Pro Gly Ala Ala Ala Ala Ala Pro Pro Ala
                405                 410                 415

Ala Arg Val Pro Asp Thr Leu Met Gln Ser Leu Ala Val Asn Trp Arg
            420                 425                 430

<210>  4
<211>  532
<212>  PRT
<213>  artificial

<220>
<223>  RTX908 polypeptide antigen

<400>  4

Lys Leu Asp Val Ile Gly Gly Asp Gly Asp Asp Val Val Leu Ala Asn
1               5                   10                  15

Ala Ser Arg Ile His Tyr Asp Gly Gly Ala Gly Thr Asn Thr Val Ser
            20                  25                  30

Tyr Ala Ala Leu Gly Arg Gln Asp Ser Ile Thr Val Ser Ala Asp Gly
        35                  40                  45

Glu Arg Phe Asn Val Arg Lys Gln Leu Asn Asn Ala Asn Val Tyr Arg
    50                  55                  60

Glu Gly Val Ala Thr Gln Thr Thr Ala Tyr Gly Lys Arg Thr Glu Asn
65                  70                  75                  80

Val Gln Tyr Arg His Val Glu Leu Ala Arg Val Gly Gln Leu Val Glu
                85                  90                  95

Val Asp Thr Leu Glu His Val Gln His Ile Ile Gly Gly Ala Gly Asn
            100                 105                 110

Asp Ser Ile Thr Gly Asn Ala His Asp Asn Phe Leu Ala Gly Gly Ser
        115                 120                 125

Gly Asp Asp Arg Leu Asp Gly Gly Ala Gly Asn Asp Thr Leu Val Gly
    130                 135                 140

Gly Glu Gly Gln Asn Thr Val Ile Gly Gly Ala Gly Asp Asp Val Phe
145                 150                 155                 160

Leu Gln Asp Leu Gly Val Trp Ser Asn Gln Leu Asp Gly Gly Ala Gly
                165                 170                 175

Val Asp Thr Val Lys Tyr Asn Val His Gln Pro Ser Glu Glu Arg Leu
            180                 185                 190

Glu Arg Met Gly Asp Thr Gly Ile His Ala Asp Leu Gln Lys Gly Thr
        195                 200                 205

Val Glu Lys Trp Pro Ala Leu Asn Leu Phe Ser Val Asp His Val Lys
    210                 215                 220

Asn Ile Glu Asn Leu His Gly Ser Arg Leu Asn Asp Arg Ile Ala Gly
225                 230                 235                 240

Asp Asp Gln Asp Asn Glu Leu Trp Gly His Asp Gly Asn Asp Thr Ile
                245                 250                 255

Arg Gly Arg Gly Gly Asp Asp Ile Leu Arg Gly Gly Leu Gly Leu Asp
            260                 265                 270

Thr Leu Tyr Gly Glu Asp Gly Asn Asp Ile Phe Leu Gln Asp Asp Glu
        275                 280                 285

Thr Val Ser Asp Asp Ile Asp Gly Gly Ala Gly Leu Asp Thr Val Asp
    290                 295                 300

Tyr Ser Ala Met Ile His Pro Gly Arg Ile Val Ala Pro His Glu Tyr
305                 310                 315                 320

Gly Phe Gly Ile Glu Ala Asp Leu Ser Arg Glu Trp Val Arg Lys Ala
                325                 330                 335

Ser Ala Leu Gly Val Asp Tyr Tyr Asp Asn Val Arg Asn Val Glu Asn
            340                 345                 350

Val Ile Gly Thr Ser Met Lys Asp Val Leu Ile Gly Asp Ala Gln Ala
        355                 360                 365

Asn Thr Leu Met Gly Gln Gly Gly Asp Asp Thr Val Arg Gly Gly Asp
    370                 375                 380

Gly Asp Asp Val Leu Leu Gly Asp Glu Gly Ser Asp Leu Leu Ser Gly
385                 390                 395                 400

Asp Ala Gly Asn Asp Asp Leu Phe Gly Gly Gln Gly Asp Asp Thr Tyr
                405                 410                 415

Leu Phe Gly Val Gly Tyr Gly His Asp Thr Ile Tyr Glu Ser Gly Gly
            420                 425                 430

Gly His Asp Thr Ile Arg Ile Asn Ala Gly Ala Asp Gln Leu Trp Phe
        435                 440                 445

Ala Arg Gln Gly Asn Asp Leu Glu Ile Arg Ile Leu Gly Thr Asp Asp
    450                 455                 460

Ala Leu Thr Val His Asp Trp Tyr Arg Asp Ala Asp His Arg Val Glu
465                 470                 475                 480

Ile Ile His Ala Ala Asn Gln Ala Val Asp Gln Ala Gly Ile Glu Lys
                485                 490                 495

Leu Val Glu Ala Met Ala Gln Tyr Pro Asp Pro Gly Ala Ala Ala Ala
            500                 505                 510

Ala Pro Pro Ala Ala Arg Val Pro Asp Thr Leu Met Gln Ser Leu Ala
        515                 520                 525

Val Asn Trp Arg
    530

<210>  5
<211>  670
<212>  PRT
<213>  artificial

<220>
<223>  RTX770 polypeptide antigen

<400>  5

Ser Ser Val Ile Gly Val Gln Thr Thr Glu Ile Ser Lys Ser Ala Leu
1               5                   10                  15

Glu Leu Ala Ala Ile Thr Gly Asn Ala Asp Asn Leu Lys Ser Val Asp
            20                  25                  30

Val Phe Val Asp Arg Phe Val Gln Gly Glu Arg Val Ala Gly Gln Pro
        35                  40                  45

Val Val Leu Asp Val Ala Ala Gly Gly Ile Asp Ile Ala Ser Arg Lys
    50                  55                  60

Gly Glu Arg Pro Ala Leu Thr Phe Ile Thr Pro Leu Ala Ala Pro Gly
65                  70                  75                  80

Glu Glu Gln Arg Arg Arg Thr Lys Thr Gly Lys Ser Glu Phe Thr Thr
                85                  90                  95

Phe Val Glu Ile Val Gly Lys Gln Asp Arg Trp Arg Ile Arg Asp Gly
            100                 105                 110

Ala Ala Asp Thr Thr Ile Asp Leu Ala Lys Val Val Ser Gln Leu Val
        115                 120                 125

Asp Ala Asn Gly Val Leu Lys His Ser Ile Lys Leu Asp Val Ile Gly
    130                 135                 140

Gly Asp Gly Asp Asp Val Val Leu Ala Asn Ala Ser Arg Ile His Tyr
145                 150                 155                 160

Asp Gly Gly Ala Gly Thr Asn Thr Val Ser Tyr Ala Ala Leu Gly Arg
                165                 170                 175

Gln Asp Ser Ile Thr Val Ser Ala Asp Gly Glu Arg Phe Asn Val Arg
            180                 185                 190

Lys Gln Leu Asn Asn Ala Asn Val Tyr Arg Glu Gly Val Ala Thr Gln
        195                 200                 205

Thr Thr Ala Tyr Gly Lys Arg Thr Glu Asn Val Gln Tyr Arg His Val
    210                 215                 220

Glu Leu Ala Arg Val Gly Gln Leu Val Glu Val Asp Thr Leu Glu His
225                 230                 235                 240

Val Gln His Ile Ile Gly Gly Ala Gly Asn Asp Ser Ile Thr Gly Asn
                245                 250                 255

Ala His Asp Asn Phe Leu Ala Gly Gly Ser Gly Asp Asp Arg Leu Asp
            260                 265                 270

Gly Gly Ala Gly Asn Asp Thr Leu Val Gly Gly Glu Gly Gln Asn Thr
        275                 280                 285

Val Ile Gly Gly Ala Gly Asp Asp Val Phe Leu Gln Asp Leu Gly Val
    290                 295                 300

Trp Ser Asn Gln Leu Asp Gly Gly Ala Gly Val Asp Thr Val Lys Tyr
305                 310                 315                 320

Asn Val His Gln Pro Ser Glu Glu Arg Leu Glu Arg Met Gly Asp Thr
                325                 330                 335

Gly Ile His Ala Asp Leu Gln Lys Gly Thr Val Glu Lys Trp Pro Ala
            340                 345                 350

Leu Asn Leu Phe Ser Val Asp His Val Lys Asn Ile Glu Asn Leu His
        355                 360                 365

Gly Ser Arg Leu Asn Asp Arg Ile Ala Gly Asp Asp Gln Asp Asn Glu
    370                 375                 380

Leu Trp Gly His Asp Gly Asn Asp Thr Ile Arg Gly Arg Gly Gly Asp
385                 390                 395                 400

Asp Ile Leu Arg Gly Gly Leu Gly Leu Asp Thr Leu Tyr Gly Glu Asp
                405                 410                 415

Gly Asn Asp Ile Phe Leu Gln Asp Asp Glu Thr Val Ser Asp Asp Ile
            420                 425                 430

Asp Gly Gly Ala Gly Leu Asp Thr Val Asp Tyr Ser Ala Met Ile His
        435                 440                 445

Pro Gly Arg Ile Val Ala Pro His Glu Tyr Gly Phe Gly Ile Glu Ala
    450                 455                 460

Asp Leu Ser Arg Glu Trp Val Arg Lys Ala Ser Ala Leu Gly Val Asp
465                 470                 475                 480

Tyr Tyr Asp Asn Val Arg Asn Val Glu Asn Val Ile Gly Thr Ser Met
                485                 490                 495

Lys Asp Val Leu Ile Gly Asp Ala Gln Ala Asn Thr Leu Met Gly Gln
            500                 505                 510

Gly Gly Asp Asp Thr Val Arg Gly Gly Asp Gly Asp Asp Val Leu Leu

515                 520                 525

Gly Asp Glu Gly Ser Asp Leu Leu Ser Gly Asp Ala Gly Asn Asp Asp
    530                 535                 540

Leu Phe Gly Gly Gln Gly Asp Asp Thr Tyr Leu Phe Gly Val Gly Tyr
545                 550                 555                 560

Gly His Asp Thr Ile Tyr Glu Ser Gly Gly Gly His Asp Thr Ile Arg
                565                 570                 575

Ile Asn Ala Gly Ala Asp Gln Leu Trp Phe Ala Arg Gln Gly Asn Asp
            580                 585                 590

Leu Glu Ile Arg Ile Leu Gly Thr Asp Asp Ala Leu Thr Val His Asp
        595                 600                 605

Trp Tyr Arg Asp Ala Asp His Arg Val Glu Ile Ile His Ala Ala Asn
    610                 615                 620

Gln Ala Val Asp Gln Ala Gly Ile Glu Lys Leu Val Glu Ala Met Ala
625                 630                 635                 640

Gln Tyr Pro Asp Pro Gly Ala Ala Ala Ala Ala Pro Pro Ala Ala Arg
                645                 650                 655

Val Pro Asp Thr Leu Met Gln Ser Leu Ala Val Asn Trp Arg
            660                 665                 670

<210>  6
<211>  569
<212>  PRT
<213>  artificial

<220>
<223>  GST-TEV-RTXm

<400>  6

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Gly Ser Thr Ser
    210                 215                 220

Gly Ser Gly His His His His His His Ser Ala Gly Leu Val Pro Arg
225                 230                 235                 240

Gly Ser Thr Ala Ile Gly Met Lys Glu Thr Ala Ala Ala Lys Phe Glu
                245                 250                 255

Arg Gln His Met Asp Ser Pro Asp Leu Gly Thr Gly Gly Gly Ser Gly
            260                 265                 270

Ile Glu Gly Arg Gly Ser Met Glu Asn Leu Tyr Phe Gln Gly Asn Ile
        275                 280                 285

Glu Asn Leu His Gly Ser Arg Leu Asn Asp Arg Ile Ala Gly Asp Asp
    290                 295                 300

Gln Asp Asn Glu Leu Trp Gly His Asp Gly Asn Asp Thr Ile Arg Gly

305                 310                 315                 320

Arg Gly Gly Asp Asp Ile Leu Arg Gly Gly Leu Gly Leu Asp Thr Leu
                325                 330                 335

Tyr Gly Glu Asp Gly Asn Asp Ile Phe Leu Gln Asp Asp Glu Thr Val
            340                 345                 350

Ser Asp Asp Ile Asp Gly Gly Ala Gly Leu Asp Thr Val Asp Tyr Ser
        355                 360                 365

Ala Met Ile His Pro Gly Arg Ile Val Ala Pro His Glu Tyr Gly Phe
    370                 375                 380

Gly Ile Glu Ala Asp Leu Ser Arg Glu Trp Val Arg Lys Ala Ser Ala
385                 390                 395                 400

Leu Gly Val Asp Tyr Tyr Asp Asn Val Arg Asn Val Glu Asn Val Ile
                405                 410                 415

Gly Thr Ser Met Lys Asp Val Leu Ile Gly Asp Ala Gln Ala Asn Thr
            420                 425                 430

Leu Met Gly Gln Gly Gly Asp Asp Thr Val Arg Gly Gly Asp Gly Asp
        435                 440                 445

Asp Val Leu Leu Gly Asp Glu Gly Ser Asp Leu Leu Ser Gly Asp Ala
    450                 455                 460

Gly Asn Asp Asp Leu Phe Gly Gly Gln Gly Asp Asp Thr Tyr Leu Phe
465                 470                 475                 480

Gly Val Gly Tyr Gly His Asp Thr Ile Tyr Glu Ser Gly Gly Gly His
                485                 490                 495

Asp Thr Ile Arg Ile Asn Ala Gly Ala Asp Gln Leu Trp Phe Ala Arg
            500                 505                 510

Gln Gly Asn Asp Leu Glu Ile Arg Ile Leu Gly Thr Asp Asp Ala Leu
        515                 520                 525

Thr Val His Asp Trp Tyr Arg Asp Ala Asp His Arg Val Glu Ile Ile
    530                 535                 540

His Ala Ala Asn Gln Ala Val Asp Gln Ala Gly Ile Glu Lys Leu Val
545                 550                 555                 560

Glu Ala Met Ala Gln Tyr Pro Asp Pro
                565

<210>  7
<211>  439
<212>  PRT
<213>  artificial

<220>
<223>  6xHis-RTX1008

<400>  7

Met His His His His His His Glu His Val Gln His Ile Ile Gly Gly
1               5                   10                  15

Ala Gly Asn Asp Ser Ile Thr Gly Asn Ala His Asp Asn Phe Leu Ala
            20                  25                  30

Gly Gly Ser Gly Asp Asp Arg Leu Asp Gly Gly Ala Gly Asn Asp Thr
        35                  40                  45

Leu Val Gly Gly Glu Gly Gln Asn Thr Val Ile Gly Gly Ala Gly Asp
    50                  55                  60

Asp Val Phe Leu Gln Asp Leu Gly Val Trp Ser Asn Gln Leu Asp Gly
65                  70                  75                  80

Gly Ala Gly Val Asp Thr Val Lys Tyr Asn Val His Gln Pro Ser Glu
                85                  90                  95

Glu Arg Leu Glu Arg Met Gly Asp Thr Gly Ile His Ala Asp Leu Gln
            100                 105                 110

Lys Gly Thr Val Glu Lys Trp Pro Ala Leu Asn Leu Phe Ser Val Asp
        115                 120                 125

His Val Lys Asn Ile Glu Asn Leu His Gly Ser Arg Leu Asn Asp Arg
    130                 135                 140

Ile Ala Gly Asp Asp Gln Asp Asn Glu Leu Trp Gly His Asp Gly Asn
145                 150                 155                 160

Asp Thr Ile Arg Gly Arg Gly Gly Asp Asp Ile Leu Arg Gly Gly Leu
                165                 170                 175

Gly Leu Asp Thr Leu Tyr Gly Glu Asp Gly Asn Asp Ile Phe Leu Gln
            180                 185                 190

Asp Asp Glu Thr Val Ser Asp Asp Ile Asp Gly Gly Ala Gly Leu Asp

195                 200                 205

Thr Val Asp Tyr Ser Ala Met Ile His Pro Gly Arg Ile Val Ala Pro
    210                 215                 220

His Glu Tyr Gly Phe Gly Ile Glu Ala Asp Leu Ser Arg Glu Trp Val
225                 230                 235                 240

Arg Lys Ala Ser Ala Leu Gly Val Asp Tyr Tyr Asp Asn Val Arg Asn
                245                 250                 255

Val Glu Asn Val Ile Gly Thr Ser Met Lys Asp Val Leu Ile Gly Asp
            260                 265                 270

Ala Gln Ala Asn Thr Leu Met Gly Gln Gly Gly Asp Asp Thr Val Arg
        275                 280                 285

Gly Gly Asp Gly Asp Asp Val Leu Leu Gly Asp Glu Gly Ser Asp Leu
    290                 295                 300

Leu Ser Gly Asp Ala Gly Asn Asp Asp Leu Phe Gly Gly Gln Gly Asp
305                 310                 315                 320

Asp Thr Tyr Leu Phe Gly Val Gly Tyr Gly His Asp Thr Ile Tyr Glu
                325                 330                 335

Ser Gly Gly Gly His Asp Thr Ile Arg Ile Asn Ala Gly Ala Asp Gln
            340                 345                 350

Leu Trp Phe Ala Arg Gln Gly Asn Asp Leu Glu Ile Arg Ile Leu Gly
        355                 360                 365

Thr Asp Asp Ala Leu Thr Val His Asp Trp Tyr Arg Asp Ala Asp His
    370                 375                 380

Arg Val Glu Ile Ile His Ala Ala Asn Gln Ala Val Asp Gln Ala Gly
385                 390                 395                 400

Ile Glu Lys Leu Val Glu Ala Met Ala Gln Tyr Pro Asp Pro Gly Ala
                405                 410                 415

Ala Ala Ala Ala Pro Pro Ala Ala Arg Val Pro Asp Thr Leu Met Gln
            420                 425                 430

Ser Leu Ala Val Asn Trp Arg
        435

<210>  8
<211>  539
<212>  PRT
<213>  artificial

<220>
<223>  6xHis-RTX908

<400>  8

Met His His His His His His Lys Leu Asp Val Ile Gly Gly Asp Gly
1               5                   10                  15

Asp Asp Val Val Leu Ala Asn Ala Ser Arg Ile His Tyr Asp Gly Gly
            20                  25                  30

Ala Gly Thr Asn Thr Val Ser Tyr Ala Ala Leu Gly Arg Gln Asp Ser
        35                  40                  45

Ile Thr Val Ser Ala Asp Gly Glu Arg Phe Asn Val Arg Lys Gln Leu
    50                  55                  60

Asn Asn Ala Asn Val Tyr Arg Glu Gly Val Ala Thr Gln Thr Thr Ala
65                  70                  75                  80

Tyr Gly Lys Arg Thr Glu Asn Val Gln Tyr Arg His Val Glu Leu Ala
                85                  90                  95

Arg Val Gly Gln Val Val Glu Val Asp Thr Leu Glu His Val Gln His
            100                 105                 110

Ile Ile Gly Gly Ala Gly Asn Asp Ser Ile Thr Gly Asn Ala His Asp
        115                 120                 125

Asn Phe Leu Ala Gly Gly Ser Gly Asp Asp Arg Leu Asp Gly Gly Ala
    130                 135                 140

Gly Asn Asp Thr Leu Val Gly Gly Glu Gly Gln Asn Thr Val Ile Gly
145                 150                 155                 160

Gly Ala Gly Asp Asp Val Phe Leu Gln Asp Leu Gly Val Trp Ser Asn
                165                 170                 175

Gln Leu Asp Gly Gly Ala Gly Val Asp Thr Val Lys Tyr Asn Val His
            180                 185                 190

Gln Pro Ser Glu Glu Arg Leu Glu Arg Met Gly Asp Thr Gly Ile His
        195                 200                 205

Ala Asp Leu Gln Lys Gly Thr Val Glu Lys Trp Pro Ala Leu Asn Leu

210                 215                 220

Phe Ser Val Asp His Val Lys Asn Ile Glu Asn Leu His Gly Ser Arg
225                 230                 235                 240

Leu Asn Asp Arg Ile Ala Gly Asp Asp Gln Asp Asn Glu Leu Trp Gly
                245                 250                 255

His Asp Gly Asn Asp Thr Ile Arg Gly Arg Gly Gly Asp Asp Ile Leu
            260                 265                 270

Arg Gly Gly Leu Gly Leu Asp Thr Leu Tyr Gly Glu Asp Gly Asn Asp
        275                 280                 285

Ile Phe Leu Gln Asp Asp Glu Thr Val Ser Asp Asp Ile Asp Gly Gly
    290                 295                 300

Ala Gly Leu Asp Thr Val Asp Tyr Ser Ala Met Ile His Pro Gly Arg
305                 310                 315                 320

Ile Val Ala Pro His Glu Tyr Gly Phe Gly Ile Glu Ala Asp Leu Ser
                325                 330                 335

Arg Glu Trp Val Arg Lys Ala Ser Ala Leu Gly Val Asp Tyr Tyr Asp
            340                 345                 350

Asn Val Arg Asn Val Glu Asn Val Ile Gly Thr Ser Met Lys Asp Val
        355                 360                 365

Leu Ile Gly Asp Ala Gln Ala Asn Thr Leu Met Gly Gln Gly Gly Asp
    370                 375                 380

Asp Thr Val Arg Gly Gly Asp Gly Asp Asp Val Leu Leu Gly Asp Glu
385                 390                 395                 400

Gly Ser Asp Leu Leu Ser Gly Asp Ala Gly Asn Asp Asp Leu Phe Gly
                405                 410                 415

Gly Gln Gly Asp Asp Thr Tyr Leu Phe Gly Val Gly Tyr Gly His Asp
            420                 425                 430

Thr Ile Tyr Glu Ser Gly Gly Gly His Asp Thr Ile Arg Ile Asn Ala
        435                 440                 445

Gly Ala Asp Gln Leu Trp Phe Ala Arg Gln Gly Asn Asp Leu Glu Ile
    450                 455                 460

Arg Ile Leu Gly Thr Asp Asp Ala Leu Thr Val His Asp Trp Tyr Arg
465                 470                 475                 480

Asp Ala Asp His Arg Val Glu Ile Ile His Ala Ala Asn Gln Ala Val
                485                 490                 495

Asp Gln Ala Gly Ile Glu Lys Leu Val Glu Ala Met Ala Gln Tyr Pro
            500                 505                 510

Asp Pro Gly Ala Ala Ala Ala Ala Pro Pro Ala Ala Arg Val Pro Asp
        515                 520                 525

Thr Leu Met Gln Ser Leu Ala Val Asn Trp Arg
    530                 535

<210>  9
<211>  677
<212>  PRT
<213>  artificial

<220>
<223>  6xHis-RTX770

<400>  9

Met His His His His His His Ser Ser Val Ile Gly Val Gln Thr Thr
1               5                   10                  15

Glu Ile Ser Lys Ser Ala Leu Glu Leu Ala Ala Ile Thr Gly Asn Ala
            20                  25                  30

Asp Asn Leu Lys Ser Val Asp Val Phe Val Asp Arg Phe Val Gln Gly
        35                  40                  45

Glu Arg Val Ala Gly Gln Pro Val Val Leu Asp Val Ala Ala Gly Gly
    50                  55                  60

Ile Asp Ile Ala Ser Arg Lys Gly Glu Arg Pro Ala Leu Thr Phe Ile
65                  70                  75                  80

Thr Pro Leu Ala Ala Pro Gly Glu Glu Gln Arg Arg Arg Thr Lys Thr
                85                  90                  95

Gly Lys Ser Glu Phe Thr Thr Phe Val Glu Ile Val Gly Lys Gln Asp
            100                 105                 110

Arg Trp Arg Ile Arg Asp Gly Ala Ala Asp Thr Thr Ile Asp Leu Ala
        115                 120                 125

Lys Val Val Ser Gln Leu Val Asp Ala Asn Gly Val Leu Lys His Ser

130                 135                 140

Ile Lys Leu Asp Val Ile Gly Gly Asp Gly Asp Asp Val Val Leu Ala
145                 150                 155                 160

Asn Ala Ser Arg Ile His Tyr Asp Gly Gly Ala Gly Thr Asn Thr Val
                165                 170                 175

Ser Tyr Ala Ala Leu Gly Arg Gln Asp Ser Ile Thr Val Ser Ala Asp
            180                 185                 190

Gly Glu Arg Phe Asn Val Arg Lys Gln Leu Asn Asn Ala Asn Val Tyr
        195                 200                 205

Arg Glu Gly Val Ala Thr Gln Thr Thr Ala Tyr Gly Lys Arg Thr Glu
    210                 215                 220

Asn Val Gln Tyr Arg His Val Glu Leu Ala Arg Val Gly Gln Val Val
225                 230                 235                 240

Glu Val Asp Thr Leu Glu His Val Gln His Ile Ile Gly Gly Ala Gly
                245                 250                 255

Asn Asp Ser Ile Thr Gly Asn Ala His Asp Asn Phe Leu Ala Gly Gly
            260                 265                 270

Ser Gly Asp Asp Arg Leu Asp Gly Gly Ala Gly Asn Asp Thr Leu Val
        275                 280                 285

Gly Gly Glu Gly Gln Asn Thr Val Ile Gly Gly Ala Gly Asp Asp Val
    290                 295                 300

Phe Leu Gln Asp Leu Gly Val Trp Ser Asn Gln Leu Asp Gly Gly Ala
305                 310                 315                 320

Gly Val Asp Thr Val Lys Tyr Asn Val His Gln Pro Ser Glu Glu Arg
                325                 330                 335

Leu Glu Arg Met Gly Asp Thr Gly Ile His Ala Asp Leu Gln Lys Gly
            340                 345                 350

Thr Val Glu Lys Trp Pro Ala Leu Asn Leu Phe Ser Val Asp His Val
        355                 360                 365

Lys Asn Ile Glu Asn Leu His Gly Ser Arg Leu Asn Asp Arg Ile Ala
    370                 375                 380

Gly Asp Asp Gln Asp Asn Glu Leu Trp Gly His Asp Gly Asn Asp Thr
385                 390                 395                 400

Ile Arg Gly Arg Gly Gly Asp Asp Ile Leu Arg Gly Gly Leu Gly Leu
                405                 410                 415

Asp Thr Leu Tyr Gly Glu Asp Gly Asn Asp Ile Phe Leu Gln Asp Asp
            420                 425                 430

Glu Thr Val Ser Asp Asp Ile Asp Gly Gly Ala Gly Leu Asp Thr Val
        435                 440                 445

Asp Tyr Ser Ala Met Ile His Pro Gly Arg Ile Val Ala Pro His Glu
    450                 455                 460

Tyr Gly Phe Gly Ile Glu Ala Asp Leu Ser Arg Glu Trp Val Arg Lys
465                 470                 475                 480

Ala Ser Ala Leu Gly Val Asp Tyr Tyr Asp Asn Val Arg Asn Val Glu
                485                 490                 495

Asn Val Ile Gly Thr Ser Met Lys Asp Val Leu Ile Gly Asp Ala Gln
            500                 505                 510

Ala Asn Thr Leu Met Gly Gln Gly Gly Asp Asp Thr Val Arg Gly Gly
        515                 520                 525

Asp Gly Asp Asp Val Leu Leu Gly Asp Glu Gly Ser Asp Leu Leu Ser
    530                 535                 540

Gly Asp Ala Gly Asn Asp Asp Leu Phe Gly Gly Gln Gly Asp Asp Thr
545                 550                 555                 560

Tyr Leu Phe Gly Val Gly Tyr Gly His Asp Thr Ile Tyr Glu Ser Gly
                565                 570                 575

Gly Gly His Asp Thr Ile Arg Ile Asn Ala Gly Ala Asp Gln Leu Trp
            580                 585                 590

Phe Ala Arg Gln Gly Asn Asp Leu Glu Ile Arg Ile Leu Gly Thr Asp
        595                 600                 605

Asp Ala Leu Thr Val His Asp Trp Tyr Arg Asp Ala Asp His Arg Val
    610                 615                 620

Glu Ile Ile His Ala Ala Asn Gln Ala Val Asp Gln Ala Gly Ile Glu
625                 630                 635                 640

Lys Leu Val Glu Ala Met Ala Gln Tyr Pro Asp Pro Gly Ala Ala Ala
                645                 650                 655

Ala Ala Pro Pro Ala Ala Arg Val Pro Asp Thr Leu Met Gln Ser Leu
            660                 665                 670

Ala Val Asn Trp Arg
        675

<210>  10
<211>  1710
<212>  DNA
<213>  artificial

<220>
<223>  GST-TEV-RTXm coding sequence

<400>  10
atgtccccta tactaggtta ttggaaaatt aagggccttg tgcaacccac tcgacttctt      60

ttggaatatc ttgaagaaaa atatgaagag catttgtatg agcgcgatga aggtgataaa     120

tggcgaaaca aaaagtttga attgggtttg gagtttccca atcttcctta ttatattgat     180

ggtgatgtta aattaacaca gtctatggcc atcatacgtt atatagctga caagcacaac     240

atgttgggtg gttgtccaaa agagcgtgca gagatttcaa tgcttgaagg agcggttttg     300

gatattagat acggtgtttc gagaattgca tatagtaaag actttgaaac tctcaaagtt     360

gattttctta gcaagctacc tgaaatgctg aaaatgttcg aagatcgttt atgtcataaa     420

acatatttaa atggtgatca tgtaacccat cctgacttca tgttgtatga cgctcttgat     480

gttgttttat acatggaccc aatgtgcctg gatgcgttcc caaaattagt ttgttttaaa     540

aaacgtattg aagctatccc acaaattgat aagtacttga aatccagcaa gtatatagca     600

tggcctttgc agggctggca agccacgttt ggtggtggcg accatcctcc aaaatcggat     660

ggttcaacta gtggttctgg tcatcaccat caccatcact ccgcgggtct ggtgccacgc     720

ggtagtactg caattggtat gaaagaaacc gctgctgcta aattcgaacg ccagcacatg     780

gacagcccag atctgggtac cggtggtggc tccggtattg agggacgcgg gtccatggaa     840

aacctgtact tccagggcaa tatcgagaat ctgcacggct cccgcctaaa cgaccgcatc     900

gccggcgacg accaggacaa cgagctctgg ggccacgatg gcaacgacac gatacgcggc     960

cggggcggcg acgacatcct gcgcggcggc ctgggcctgg acacgctgta tggcgaggac    1020

ggcaacgaca tcttcctgca ggacgacgag accgtcagcg atgacatcga cggcggcgcg    1080

gggctggaca ccgtcgacta ctccgccatg atccatccag gcaggatcgt tgcgccgcat    1140

gaatacggct tcgggatcga ggcggacctg tccagggaat gggtgcgcaa ggcgtccgcg    1200

ctgggcgtgg actattacga taatgtccgc aatgtcgaaa acgtcatcgg tacgagcatg    1260

aaggatgtgc tcatcggcga cgcgcaagcc aataccctga tgggccaggg cggcgacgat 1320

accgtgcgcg gcggcgacgg cgatgacgtc ctgctgggcg acgagggctc ggacctgctc 1380

agcggcgatg cgggcaacga cgatctgttc ggcgggcagg gcgatgatac ttatctgttc 1440

ggggtcgggt acgggcacga cacgatctac gaatcgggcg gcggccatga caccatccgc 1500

atcaacgcgg gggcggacca gctgtggttc gcgcgccagg gcaacgacct ggagatccgc 1560

attctcggca ccgacgatgc acttaccgtg cacgactggt atcgcgacgc cgatcaccgg 1620

gtggaaatca tccatgccgc caaccaggcg gtagaccagg caggcatcga aaagctggtc 1680

gaggcaatgg cgcagtatcc ggacccctga 1710

<210> 11
<211> 1320
<212> DNA
<213> artificial

<220>
<223> 6xHis-RTX1008 coding sequence

<400> 11
atgcatcatc atcatcatca tgagcatgtg cagcacatca tcggcggggc cggcaacgat 60

tcgatcaccg gcaatgcgca cgacaacttc ctagccggcg ggtcgggcga cgacaggctg 120

gatggcggcg ccggcaacga caccctggtt ggcggcgagg gccaaaacac ggtcatcggc 180

ggcgccggcg acgacgtatt cctgcaggac ctgggggtat ggagcaacca gctcgatggc 240

ggcgcgggcg tcgataccgt gaagtacaac gtgcaccagc cttccgagga gcgcctcgaa 300

cgcatgggcg acacgggcat ccatgccgat cttcaaaagg gcacggtcga gaagtggccg 360

gccctgaacc tgttcagcgt cgaccatgtc aagaatatcg agaatctgca cggctcccgc 420

ctaaacgacc gcatcgccgg cgacgaccag gacaacgagc tctgggccca cgatggcaac 480

gacacgatac gcggccgggg cggcgacgac atcctgcgcg gcggcctggg cctggacacg 540

ctgtatggcg aggacggcaa cgacatcttc ctgcaggacg acgagaccgt cagcgatgac 600

atcgacggcg gcgcggggct ggacaccgtc gactactccg ccatgatcca tccaggcagg 660

atcgttgcgc cgcatgaata cggcttcggg atcgaggcgg acctgtccag ggaatgggtg 720

cgcaaggcgt ccgcgctggg cgtggactat tacgataatg tccgcaatgt cgaaaacgtc 780

atcggtacga gcatgaagga tgtgctcatc ggcgacgcgc aagccaatac cctgatgggc 840

cagggcggcg acgataccgt gcgcggcggc gacggcgatg acgtcctgct gggcgacgag 900

ggctcggacc tgctcagcgg cgatgcgggc aacgacgatc tgttcggcgg gcagggcgat 960

gatacttatc tgttcggggt cgggtacggg cacgacacga tctacgaatc gggcggcggc 1020

catgacacca tccgcatcaa cgcgggggcg gaccagctgt ggttcgcgcg ccagggcaac 1080

gacctggaga tccgcattct cggcaccgac gatgcactta ccgtgcacga ctggtatcgc 1140

gacgccgatc accgggtgga aatcatccat gccgccaacc aggcggtaga ccaggcaggc 1200

atcgaaaagc tggtcgaggc aatggcgcag tatccggacc ccggcgcggc ggcggctgcc 1260

ccgccggcgg cgcgcgtgcc ggacacgctg atgcagtccc tggctgtcaa ctggcgctga 1320

<210> 12
<211> 1620
<212> DNA
<213> artificial

<220>
<223> 6xHis-RTX908 coding sequence

<400> 12
atgcatcatc atcatcatca taaactggat gtgatcggcg gagatggcga tgacgtcgtg 60

cttgccaatg cttcgcgcat ccattatgac ggcggcgcgg gcaccaacac ggtcagctat 120

gccgccctgg gtcgacagga ttccattacc gtgtccgccg acggggaacg tttcaacgtg 180

cgcaagcagt tgaacaacgc caacgtgtat cgcgaaggcg tggctaccca gacaaccgcc 240

tacggcaagc gcacggagaa tgtccaatac cgccatgtcg agctggcccg tgtcgggcaa 300

gtggtggagg tcgacacgct cgagcatgtg cagcacatca tcggcggggc cggcaacgat 360

tcgatcaccg gcaatgcgca cgacaacttc ctagccggcg ggtcgggcga cgacaggctg 420

gatggcggcg ccggcaacga caccctggtt ggcggcgagg gccaaaacac ggtcatcggc 480

ggcgccggcg acgacgtatt cctgcaggac ctgggggtat ggagcaacca gctcgatggc 540

ggcgcgggcg tcgataccgt gaagtacaac gtgcaccagc cttccgagga gcgcctcgaa 600

cgcatgggcg acacgggcat ccatgccgat cttcaaaagg gcacggtcga gaagtggccg 660

gccctgaacc tgttcagcgt cgaccatgtc aagaatatcg agaatctgca cggctcccgc 720

ctaaacgacc gcatcgccgg cgacgaccag gacaacgagc tctgggccca cgatggcaac 780

gacacgatac gcggccgggg cggcgacgac atcctgcgcg gcggcctggg cctggacacg 840

ctgtatggcg aggacggcaa cgacatcttc ctgcaggacg acgagaccgt cagcgatgac 900

atcgacggcg gcgcggggct ggacaccgtc gactactccg ccatgatcca tccaggcagg 960

atcgttgcgc cgcatgaata cggcttcggg atcgaggcgg acctgtccag ggaatgggtg 1020

cgcaaggcgt ccgcgctggg cgtggactat tacgataatg tccgcaatgt cgaaaacgtc 1080

atcggtacga gcatgaagga tgtgctcatc ggcgacgcgc aagccaatac cctgatgggc 1140

cagggcggcg acgataccgt gcgcggcggc gacggcgatg acgtcctgct gggcgacgag 1200

ggctcggacc tgctcagcgg cgatgcgggc aacgacgatc tgttcggcgg gcagggcgat 1260

gatacttatc tgttcggggt cgggtacggg cacgacacga tctacgaatc gggcggcggc 1320

catgacacca tccgcatcaa cgcgggggcg gaccagctgt ggttcgcgcg ccagggcaac 1380

gacctggaga tccgcattct cggcaccgac gatgcactta ccgtgcacga ctggtatcgc 1440

gacgccgatc accgggtgga aatcatccat gccgccaacc aggcggtaga ccaggcaggc 1500

atcgaaaagc tggtcgaggc aatggcgcag tatccggacc ccggcgcggc ggcggctgcc 1560

ccgccggcgg cgcgcgtgcc ggacacgctg atgcagtccc tggctgtcaa ctggcgctga 1620

<210> 13
<211> 2034
<212> DNA
<213> artificial

<220>
<223> 6xHis-RTX770 coding sequence

<400> 13
atgcatcatc atcatcatca tagcagcgtg atcggggtgc agacgacaga gatctccaag 60

tcggcgctcg aactggccgc cattaccggc aacgcggaca acctgaaatc cgtcgacgtg 120

ttcgtggacc gcttcgtcca gggcgagcgg gtggccggcc agccggtggt cctcgacgtc 180

gccgccggcg gcatcgatat cgccagccgc aagggcgagc ggccggcgct gacgttcatc 240

acgccgctgg ccgcgccagg agaagagcag cgccggcgca cgaaaacggg caagagcgaa 300

ttcaccacat tcgtcgagat cgtgggcaag caggaccgct ggcgcatccg ggacggcgcg 360

gccgacacca ccatcgatct ggccaaggtg gtgtcgcaac tggtcgacgc caatggcgtg 420

ctcaagcaca gcatcaaact ggatgtgatc ggcggagatg gcgatgacgt cgtgcttgcc 480

aatgcttcgc gcatccatta tgacggcggc gcgggcacca acacggtcag ctatgccgcc 540

ctgggtcgac aggattccat taccgtgtcc gccgacgggg aacgtttcaa cgtgcgcaag 600

cagttgaaca acgccaacgt gtatcgcgaa ggcgtggcta cccagacaac cgcctacggc 660

aagcgcacgg agaatgtcca ataccgccat gtcgagctgg cccgtgtcgg gcaagtggtg 720

gaggtcgaca cgctcgagca tgtgcagcac atcatcggcg gggccggcaa cgattcgatc 780

accggcaatg cgcacgacaa cttcctagcc ggcgggtcgg gcgacgacag gctggatggc 840

ggcgccggca acgacaccct ggttggcggc gagggccaaa acacggtcat cggcggcgcc 900

ggcgacgacg tattcctgca ggacctgggg gtatggagca accagctcga tggcggcgcg 960

ggcgtcgata ccgtgaagta caacgtgcac cagccttccg aggagcgcct cgaacgcatg 1020

ggcgacacgg gcatccatgc cgatcttcaa aagggcacgg tcgagaagtg gccggccctg 1080

aacctgttca gcgtcgacca tgtcaagaat atcgagaatc tgcacggctc ccgcctaaac 1140

gaccgcatcg ccggcgacga ccaggacaac gagctctggg gccacgatgg caacgacacg 1200

atacgcggcc ggggcggcga cgacatcctg cgcggcggcc tgggcctgga cacgctgtat 1260

ggcgaggacg gcaacgacat cttcctgcag gacgacgaga ccgtcagcga tgacatcgac 1320

ggcggcgcgg ggctggacac cgtcgactac tccgccatga tccatccagg caggatcgtt 1380

gcgccgcatg aatacggctt cgggatcgag gcggacctgt ccagggaatg ggtgcgcaag 1440

gcgtccgcgc tgggcgtgga ctattacgat aatgtccgca atgtcgaaaa cgtcatcggt 1500

acgagcatga aggatgtgct catcggcgac gcgcaagcca ataccctgat gggccagggc 1560

ggcgacgata ccgtgcgcgg cggcgacggc gatgacgtcc tgctgggcga cgagggctcg 1620

gacctgctca gcggcgatgc gggcaacgac gatctgttcg gcgggcaggg cgatgatact 1680

tatctgttcg gggtcgggta cgggcacgac acgatctacg aatcgggcgg cggccatgac 1740

accatccgca tcaacgcggg ggcggaccag ctgtggttcg cgcgccaggg caacgacctg 1800

gagatccgca ttctcggcac cgacgatgca cttaccgtgc acgactggta tcgcgacgcc 1860

gatcaccggg tggaaatcat ccatgccgcc aaccaggcgg tagaccaggc aggcatcgaa 1920

aagctggtcg aggcaatggc gcagtatccg gaccccggcg cggcggcggc tgccccgccg 1980

gcggcgcgcg tgccggacac gctgatgcag tccctggctg tcaactggcg ctga 2034

## Description

### Field of Art

The present invention relates to a recombinant polypeptide antigen derived from the Adenylate cyclase toxin (ACT, AC-Hly or CyaA) of *Bordetella pertussis.*

### Background Art

*Bordetella pertussis,* the causative agent of whooping cough, secretes an adenylate cyclase toxin-hemolysin (CyaA, ACT or AC-Hly), a member of the Repeat-in-ToXin (RTX) protein family that plays a crucial role in the early stages of bacterial colonization of human respiratory tract. CyaA targets and annihilates bactericidal activities of phagocytes, such as oxidative burst and complement- or antibody-mediated opsonophagocytic uptake and killing of bacteria by neutrophils and macrophages. Through cAMP signaling, CyaA skews also TLR-triggered maturation of dendritic cells, inhibiting pro-inflammatory IL-12 and TNF-α secretion and enhancing IL-10 production and Treg expansion, likely hampering also induction of adaptive immune responses to *Bordetella* infections.

Action of the adenylate cyclase toxin, playing a critical role in the early phases of bacterial colonization, appears to be of crucial importance for suppression of host innate immune mechanisms, as well as for modulation of adaptive T and B cell responses and for evasion of host immunity. The absence of CyaA in the current acellular pertussis (aP) vaccine formulation and the failure of such vaccines to induce CyaA-neutralizing antibodies represents one of the key factors contributing to the failure of the aP vaccines in conferring a long-lasting immune protection against whooping cough in highly vaccinated populations. There is an urgent public health need to improve the composition of the aP vaccine so as to enhance the protection of aP-vaccinated subjects against colonization by the pathogen and block its transmission that gives rise to pertussis resurgence in the most developed countries. Given the protective immunogenicity of CyaA and its major immunosuppressive role played in *Bordetella* infections, CyaA toxoids are a prime candidate for inclusion into the next generation of improved aP vaccines.

CyaA is a 1,706-residue-long (177-kDa) bifunctional protein that consists of an amino-terminal adenylate cyclase (AC) domain of about 400 residues and of an RTX hemolysin moiety (Hly) of about 1,300 residues. The Hly moiety inserts into cellular membranes and mediates translocation of the enzymatic AC domain into cell cytosol, where this binds calmodulin and catalyzes conversion of ATP to cAMP, thus subverting cellular signaling. In parallel, the Hly can form small cation-selective membrane pores that permeabilize target cell membrane and allow efflux of potassium ions from cells, thus causing colloid-osmotic cell lysis, such as hemolysis of erythrocytes.

The capacity of CyaA to tightly associate with the cell membrane of myeloid phagocytic cells and translocate the AC enzyme domain into these cells to neutralize their bactericidal capacities by elevation of cAMP concentration depends on toxin binding to the complement receptor 3 on the surface of phagocytes (CR3, or the heterodimeric α_{M}β₂ integrin CD11b/CD18, or Mac-1). This occurs through a segment of the RTX domain roughly delimited by residues 1166 and 1287 of CyaA (Guermonprez, et al. 2001, J Exp Med 193, pp. 1035-1044; El Azami El Idrisi et al. 2003 JBC 278, pp. 38514-38521; Osicka et al. 2015 Elife 4: e10766).

CyaA-deficient *Bordetella* strains were shown to be significantly compromised in their host colonization capacity and in persistence in various mouse models (Carbonetti et al. 2005, Infect. Immun. 73(5), pp.2698-2703; Goodwin, M., and Weiss, A. 1990, Infect. Immun. 58, 3445-3447; Khelef et al. 1992, Microb. Pathog. 12, pp.227-235). Some hypervirulent strains of *Bordetella pertussis* express higher CyaA levels (Guiso et al. 1989, Microb. Pathog.7, pp. 373-380). Moreover, active or passive immunization with polyclonal anti-CyaA antibodies protected mice against lethal respiratory challenges by *B. pertussis* and *B. parapertussis* (Guiso et al. 1989, Microb. Pathog.7, pp. 373-380) and shortened the period of bacterial colonization in the respiratory tract (Guiso et al. 1991, Microb. Pathog. 11, pp.423-431). Finally, natural infection of humans results in a strong anti-CyaA antibody response (Farfel et al. 1990, J. Med. Microbiol 32, pp. 173-177) and such antibodies in most of sera from convalescent patients are potently neutralizing the toxin activity of CyaA towards phagocytic cells (Eby et al. 2017, Clin. Vaccine Immunol. 24(1), pii: e00370-16. doi: 10.1128/CVI.00370-16). Previously, various truncated variants of CyaA, generated by deletions of its segments were shown to be recognized in immunoblots by sera of infected mice and humans, providing that the RTX domain segment was intact (Betsou et al. 1995, Infect. Immun. 63, pp. 3309-3315). A construct lacking only the last 217 out of 1706 residues of CyaA, however, did not confer any protective immunity, nor induced any toxin-neutralizing antibodies, presumably due to lack of capacity to fold properly (Bumba et al. 2016, Mol. Cell 62, pp. 47-62) and present the conformational epitopes of the RTX domain formed by the structure of RTX blocks II and III and recognized by toxin-neutralizing monoclonal antibodies (WO2016094438A1; Wang. et al. 2017, Biochemistry 56, pp. 1324-1336). This highlighted the importance of the C-terminal region for AC toxin structure and activity. However, a protein lacking residues 385-1489, but bearing the last 217 residues could still induce antibodies neutralizing toxin activity towards non-myeloid cells lacking the CR3 receptor *in vitro*, but failed to confer protective immunity *in vivo*, presumably by failing to present the conformational epitopes needed for induction of antibodies neutralizing CyaA binding to the CR3 receptor of myeloid phagocytic cells and the paralyzing activity of CyaA on such cells (Confer D. L. and Eaton J.W. 1982, Science 217, pp. 948-950; Cerny et al. 2017, J. Immunol. 198, pp. 1285-1296) .

### Disclosure of the Invention

The present invention relates to a novel type of recombinant and CyaA-derived polypeptide antigens of less than 73 kDa in size, which were assembled by fusing at least segments of the RTX blocks II and III with a portion of the RTX block V of CyaA. These polypeptide antigens were then engineered so as to exhibit the capacity to bind calcium ions and fold in their presence into a structure that presents on its surface the epitopes inducing CyaA toxin-neutralizing antibodies *in vivo*. Prior art teaching suggested that the presence of the whole RTX domain is needed for maintaining the ability of the CyaA-derived antigens to induce CyaA toxin-neutralizing antibodies *in vivo.* The present invention surprisingly overcomes this teaching.

The present invention thus provides a polypeptide antigen derived from amino acid sequence of adenylate cyclase protein (CyaA, SEQ ID NO. 1) of *Bordetella pertussis*, comprising a polypeptide sequence consisting of:
(i) amino acid sequence of a fragment of the RTX blocks II and III of CyaA, having on its N-terminus an amino acid residue in position between 1132-1166 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681,
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

More preferably, the polypeptide antigen comprises a polypeptide sequence consisting of:
(i) amino acid sequence of residues 1166 to 1287, or 1140 to 1290, or 1132 to 1294 of CyaA, or , and to its C-terminus fused
(ii) amino acid sequence of residues 1570 to 1680, or 1570 to 1681, or 1562 to 1680, or 1562 to 1681, or 1565 to 1680, or 1565 to 1681 of CyaA,
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

In some embodiments, the polypeptide antigen derived from amino acid sequence of adenylate cyclase protein (CyaA, SEQ ID NO. 1) of *Bordetella pertussis,* comprises a polypeptide sequence consisting of:
(i) amino acid sequence of a fragment of the RTX blocks I, II and III of CyaA, having on its N-terminus an amino acid residue in position between 984-1008 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681;
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

In some embodiments, the polypeptide antigen derived from amino acid sequence of adenylate cyclase protein (CyaA, SEQ ID NO. 1) of *Bordetella pertussis*, comprises a polypeptide sequence consisting of:
(i) amino acid sequence of a fragment of the RTX blocks I, II and III of CyaA, having on its N-terminus an amino acid residue in position between 905-915 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681;
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

In some embodiments, the polypeptide antigen derived from amino acid sequence of adenylate cyclase protein (CyaA, SEQ ID NO. 1) of *Bordetella pertussis*, comprises a polypeptide sequence consisting of:
(i) amino acid sequence of a fragment of the RTX blocks I, II and III of CyaA, having on its N-terminus an amino acid residue in position between 765-774 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681;
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

By a fragment of CyaA, having on its N-terminus an amino acid residue with a defined position and having on its C-terminus an amino acid residue with a defined position, the portion of the sequence of CyaA of *Bordetella pertussis* extending between the said N-terminal and the said C-terminal amino acids should be understood.

CyaA has been described as an amino acid sequence, encoded by the *cyaA* gene (BP0760 of GenBank: BX470248.1, Gene ID: 2664492, Sequence: NC_002929.2 (776228..781348)). Accordingly, when amino acid residues or sequences or fragments thereof, or nucleotides or nucleotide sequences of the CyaA protein of *B. pertussis* or of the *cyaA* gene are cited in the present invention, their positions are given with respect to the sequence of wild-type CyaA protein, where "CyaA protein" or "wild-type CyaA protein" or "CyaA" refers to the amino acid residue sequence of adenylate cyclase toxin protein from *Bordetella pertussis* deposited at UniProtKB/Swiss-Prot database under Acc. No.: P0DKX7.1 (https://www.ncbi.nlm.nih.gov/protein/P0DKX7.1) and shown herein as SEQ ID NO. 1.
SEQ ID NO. 1 - wild-type CyaA protein of *B. pertussis*

1 MQQSHQAGYA NAADRESGIP AAVLDGIKAV AKEKNATLMF RLVNPHSTSL IAEGVATKGL
61 GVHAKSSDWG LQAGYIPVNP NLSKLFGRAP EVIARADNDV NSSLAHGHTA VDLTLSKERL
121 DYLRQAGLVT GMADGVVASN HAGYEQFEFR VKETSDGRYA VQYRRKGGDD FEAVKVIGNA
181 AGIPLTADID MFAIMPHLSN FRDSARSSVT SGDSVTDYLA RTRRAASEAT GGLDRERIDL
241 LWKIARAGAR SAVGTEARRQ FRYDGDMNIG VITDFELEVR NALNRRAHAV GAQDVVQHGT
301 EQNNPFPEAD EKIFVVSATG ESQMLTRGQL KEYIGQQRGE GYVFYENRAY GVAGKSLFDD
361 GLGAAPGVPS GRSKFSPDVL ETVPASPGLR RPSLGAVERQ DSGYDSLDGV GSRSFSLGEV
421 SDMAAVEAAE LEMTRQVLHA GARQDDAEPG VSGASAHWGQ RALQGAQAVA AAQRLVHAIA
481 LMTQFGRAGS TNTPQEAASL SAAVFGLGEA SSAVAETVSG FFRGSSRWAG GFGVAGGAMA
541 LGGGIAAAVG AGMSLTDDAP AGQKAAAGAE IALQLTGGTV ELASSIALAL AAARGVTSGL
601 QVAGASAGAA AGALAAALSP MEIYGLVQQS HYADQLDKLA QESSAYGYEG DALLAQLYRD
661 KTAAEGAVAG VSAVLSTVGA AVSIAAAASV VGAPVAVVTS LLTGALNGIL RGVQQPIIEK
721 LANDYARKID ELGGPQAYFE KNLQARHEQL ANSDGLRKML ADLQAGWNAS SVIGVQTTEI
781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDDLLFGGD GNDMLYGDAG NDTLYGGLGD
1321 DTLEGGAGND WFGQTQAREH DVLRGGDGVD TVDYSQTGAH AGIAAGRIGL GILADLGAGR
1381 VDKLGEAGSS AYDTVSGIEN VVGTELADRI TGDAQANVLR GAGGADVLAG GEGDDVLLGG
1441 DGDDQLSGDA GRDRLYGEAG DDWFFQDAAN AGNLLDGGDG RDTVDFSGPG RGLDAGAKGV
1501 FLSLGKGFAS LMDEPETSNV LRNIENAVGS ARDDVLIGDA GANVLNGLAG NDVLSGGAGD
1561 DVLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR

The amino acid sequence corresponding to a fragment of RTX block V (flanked by its C-terminal capping structure) is the necessary part of the structure that triggers and scaffolds vectorial folding of the RTX β-roll structure of block V in the presence of calcium ions (Bumba et al. 2016, Mol. Cell 62, pp. 47-62).

The site on the toxin molecule that binds to the receptor was localized between residues 1166 and 1287 (El Azami El Idrisi et al. 2003 JBC 278, pp. 38514-38521) and the negatively charged residues at positions 1193, 1194, 1232 and 1234 were implicated in electrostatic interaction with positively charged residues within the CyaA-binding structure on the CD11b subunit of the CR3 receptor (Osicka et al. 2015 Elife 4: e10766).

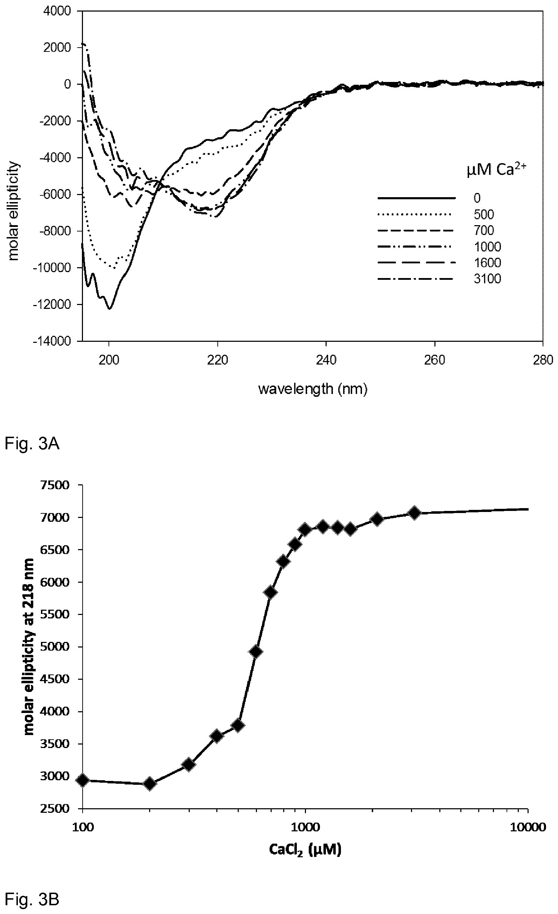

The polypeptide antigen of the present invention comprises an assembly of selected segments of blocks II, III and V of the RTX domain, comprised within residues 1132 to 1294 and residues 1561 or 1562 to 1681. In the framework of the present invention, we have demonstrated that the said assembly of CyaA segments can undergo calcium-dependent folding (Fig. 3) into a structure capable to induce CyaA-neutralizing antibodies *in vivo*. Prior art did not provide any such assembly of segments allowing to maintain the antibody-inducing activity while effectively minimizing the size of the polypeptide antigen. The polypeptide antigen is soluble, and allows to increase the concentration of protective epitopes when formulated into vaccine (compared to CyaA, it allows to provide up to 5x higher molar concentration at the same total protein amount).

As used herein, the expression "RTX domain" refers to the about 699 amino acid residue sequence following the pore-forming and acylated domain segments of the CyaA protein, preferably it refers to the amino acid residues 1006 to 1706.

The polypeptide antigen of the present invention may have the segment of repeat blocks II to III fused to C-terminal segments of CyaA, comprising fragments of the block V of various lengths. Preferably, the sequence derived from the CyaA protein comprises the amino acid CyaA sequence fragment 1166-1287, or 1140-1290, or 1132-1294, and the amino acid CyaA sequence fragment 1562 to 1691, 1562 to 1690, 1562 to 1700, 1562 to 1706, or 1561 to 1706.

In a particularly preferred embodiment, the present invention provides polypeptide antigens comprising an amino acid sequence SEQ ID NO. 2.
SEQ ID NO. 2 (amino acids 1132-1294 and 1562-1681 of CyaA) - herein referred to as RTXm

1132                                                                NIENLHGSR
1141  LNDRIAGDDQ DNELWGHDGN DTIRGRGGDD ILRGGLGLDT LYGEDGNDIF LQDDETVSDD
1201  IDGGAGLDTV DYSAMIHPGR IVAPHEYGFG IEADLSREWV RKASALGVDY YDNVRNVENV
1261  IGTSMKDVLI GDAQANTLMG QGGDDTVRGG DGDD
1562   VLLGDEGSD LLSGDAGNDD LFGGQGDDTY LFGVGYGHDT IYESGGGHDT IRINAGADQL
1621  WFARQGNDLE IRILGTDDAL TVHDWYRDAD HRVEIIHAAN QAVDQAGIEK LVEAMAQYPD
1681  P

In some embodiments, the polypeptide antigens are to be used in the absence of free Ca²⁺ ions, and it is advantageous to develop a more stable RTX structure, resistant against denaturation/unfolding and against the loss of neutralizing epitopes in the absence of free Ca²⁺ ions. Furthermore, achieving the stabilization of the interaction with aluminium vaccine adjuvants (e.g., aluminium hydroxide or aluminium phosphate) results in a higher immunogenicity. These effects can be achieved by extending the polypeptide antigen sequence corresponding to parts of RTX blocks II and III to include the RTX block I, i.e., the sequence is extended on its N-terminus by the amino acids 1008-1131 of the CyaA sequence. In some embodiments, the polypeptide antigen sequence is extended on its N-terminus by the amino acids 908-1007 and 1008-1131 of the CyaA sequence. This part of the sequence contains lysine K983, the acylation of which is essential for strong binding into cell membrane. In some embodiments, the polypeptide antigen sequence is extended on its N-terminus by the amino acids 770-1007 and 1008-1131 of the CyaA sequence. This part of the sequence contains lysines K860 and K983, which can be acylated to achieve strong binding into cell membrane. The polypeptide antigens according to these embodiments are particularly suitable for use in vaccines which require the presence of aluminium adjuvants and/or the absence of calcium ions, e.g., in vaccines for peadriatric use, or vaccines for maternal immunization and/or booster vaccines.

The polypeptide antigens may optionally contain the C-terminal part of the CyaA protein (amino acids in positions 1681-2 to 1706), to enable the secretion of the recombinant products from bacterial host cells.

In particularly preferred embodiments, the polypeptide antigens comprise a sequence selected from the following sequences:
SEQ ID NO.3 (amino acids 1008-1294 and 1562-1706 of CyaA)-herein referred to as RTX1008 
1008                                                    EHV QHIIGGAGND
1021  SITGNAHDNF LAGGSGDDRL DGGAGNDTLV GGEGQNTVIG GAGDDVFLQD LGVWSNQLDG
1081  GAGVDTVKYN VHQPSEERLE RMGDTGIHAD LQKGTVEKWP ALNLFSVDHV KNIENLHGSR
1141  LNDRIAGDDQ DNELWGHDGN DTIRGRGGDD ILRGGLGLDT LYGEDGNDIF LQDDETVSDD
1201  IDGGAGLDTV DYSAMIHPGR IVAPHEYGFG IEADLSREWV RKASALGVDY YDNVRNVENV
1261  IGTSMKDVLI GDAQANTLMG QGGDDTVRGG DGDD
1562   VLLGDEGSD LLSGDAGNDD LFGGQGDDTY LFGVGYGHDT IYESGGGHDT IRINAGADQL
1621  WFARQGNDLE IRILGTDDAL TVHDWYRDAD HRVEIIHAAN QAVDQAGIEK LVEAMAQYPD
1681  PGAAAAAPPA ARVPDTLMQS LAVNWR
SEQ ID NO. 4 (amino acids 908-1294 and 1562-1706 of CyaA) - herein referred to as RTX908 
908 KLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDD
1562 VLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR
SEQ ID NO. 5 (amino acids 770-1294 and 1562-1681 of CyaA) - herein referred to as RTX770

770 S SVIGVQTTEI
781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDD
1562 VLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR

In a second aspect, the present invention relates to a chimeric protein comprising the polypeptide antigen as defined above that is fused to a heterologous polypeptide. The heterologous polypeptide is preferably fused to the N-terminus of the polypeptide antigen.

Thus, the polypeptide antigens of the invention may be further combined with non-CyaA molecules, thereby giving rise to chimeric proteins, wherein said polypeptide antigens or chimeric proteins have a prophylactic vaccinating and/or therapeutic effect when administered to a human.

The invention thus also relates to a chimeric protein (a derivative of the polypeptide antigen of the invention) which comprises the CyaA-derived polypeptide antigen according to the invention recombined to one or more molecules, in particular to one or more molecules suitable for eliciting an immune response, thus constituting a recombinant or fusion or chimeric protein. The invention also relates to chimeric proteins obtained by chemically grafting said molecule(s) to the polypeptide antigens.

In one embodiment, the present invention relates to a chimeric protein, wherein the heterologous polypeptide comprises an affinity tag and a cleavage site. Such chimeric protein is suitable for use in affinity purification of the polypeptide antigen of the invention and subsequent cleavage of the affinity tag. An example of such chimeric protein may be a protein GST-TEV-polypeptide antigen (example: SEQ ID NO. 6), i.e., a chimeric protein comprising a Glutathione S-transferase (GST) moiety used in affinity purification of the polypeptide antigen, a sequence of a TEV site for cleavage by the TEV protease from the Tobacco Etch Virus (TEV), and the polypeptide antigen of the present invention. Another example of such chimeric protein may be a protein 6xHis-polypeptide antigen (examples : SEQ ID NO. 7, 8 and 9), i.e., a chimeric protein comprising a 6 x His moiety used in affinity purification of the polypeptide antigen fused to the N-terminus of the polypeptide antigen of the present invention.
SEQ ID NO. 6 - amino acid sequence of the GST-TEV-blockII-III/V protein used in production of the RTXm polypeptide antigen of the present invention 
1 MSPILGYWKI KGLVQPTRLL LEYLEEKYEE HLYERDEGDK WRNKKFELGL EFPNLPYYID
61 GDVKLTQSMA IIRYIADKHN MLGGCPKERA EISMLEGAVL DIRYGVSRIA YSKDFETLKV
121 DFLSKLPEML KMFEDRLCHK TYLNGDHVTH PDFMLYDALD VVLYMDPMCL DAFPKLVCFK
181 KRIEAIPQID KYLKSSKYIA WPLQGWQATF GGGDHPPKSD GSTSGSGHHH HHHSAGLVPR
241 GSTAIGMKET AAAKFERQHM DSPDLGTGGG SGIEGRGSME NLYFQGNIEN LHGSRLNDRI
301 AGDDQDNELW GHDGNDTIRG RGGDDILRGG LGLDTLYGED GNDIFLQDDE TVSDDIDGGA
361 GLDTVDYSAM IHPGRIVAPH EYGFGIEADL SREWVRKASA LGVDYYDNVR NVENVIGTSM
421 KDVLIGDAQA NTLMGQGGDD TVRGGDGDDV LLGDEGSDLL SGDAGNDDLF GGQGDDTYLF
481 GVGYGHDTIY ESGGGHDTIR INAGADQLWF ARQGNDLEIR ILGTDDALTV HDWYRDADHR
541 VEIIHAANQA VDQAGIEKLV EAMAQYPDP
SEQ ID NO. 7 - amino acid sequence of the 6xHis-RTX1008 protein used in production of the RTX1008 polypeptide antigen of the present invention 
1       MHHHHHHEHV QHIIGGAGND SITGNAHDNF LAGGSGDDRL DGGAGNDTLV GGEGQNTVIG
61      GAGDDVFLQD LGVWSNQLDG GAGVDTVKYN VHQPSEERLE RMGDTGIHAD LQKGTVEKWP
121     ALNLFSVDHV KNIENLHGSR LNDRIAGDDQ DNELWGHDGN DTIRGRGGDD ILRGGLGLDT
181     LYGEDGNDIF LQDDETVSDD IDGGAGLDTV DYSAMIHPGR IVAPHEYGFG IEADLSREWV
241     RKASALGVDY YDNVRNVENV IGTSMKDVLI GDAQANTLMG QGGDDTVRGG DGDDVLLGDE
301     GSDLLSGDAG NDDLFGGQGD DTYLFGVGYG HDTIYESGGG HDTIRINAGA DQLWFARQGN
361     DLEIRILGTD DALTVHDWYR DADHRVEIIH AANQAVDQAG IEKLVEAMAQ YPDPGAAAAA
421     PPAARVPDTL MQSLAVNWR
SEQ ID NO. 8 - amino acid sequence of the 6xHis-RTX908 protein used in production of the RTX908 polypeptide antigen of the present invention 
1       MHHHHHHKLD VIGGDGDDVV LANASRIHYD GGAGTNTVSY AALGRQDSIT VSADGERFNV
61      RKQLNNANVY REGVATQTTA YGKRTENVQY RHVELARVGQ VVEVDTLEHV QHIIGGAGND
121     SITGNAHDNF LAGGSGDDRL DGGAGNDTLV GGEGQNTVIG GAGDDVFLQD LGVWSNQLDG
181     GAGVDTVKYN VHQPSEERLE RMGDTGIHAD LQKGTVEKWP ALNLFSVDHV KNIENLHGSR
241     LNDRIAGDDQ DNELWGHDGN DTIRGRGGDD ILRGGLGLDT LYGEDGNDIF LQDDETVSDD
301     IDGGAGLDTV DYSAMIHPGR IVAPHEYGFG IEADLSREWV RKASALGVDY YDNVRNVENV
361     IGTSMKDVLI GDAQANTLMG QGGDDTVRGG DGDDVLLGDE GSDLLSGDAG NDDLFGGQGD
421     DTYLFGVGYG HDTIYESGGG HDTIRINAGA DQLWFARQGN DLEIRILGTD DALTVHDWYR
481     DADHRVEIIH AANQAVDQAG IEKLVEAMAQ YPDPGAAAAA PPAARVPDTL MQSLAVNWR
SEQ ID NO. 9 - amino acid sequence of the 6xHis-RTX770 protein used in production of the RTX770 polypeptide antigen of the present invention

1 MHHHHHHSSV IGVQTTEISK SALELAAITG NADNLKSVDV FVDRFVQGER VAGQPVVLDV
61 AAGGIDIASR KGERPALTFI TPLAAPGEEQ RRRTKTGKSE FTTFVEIVGK QDRWRIRDGA
121 ADTTIDLAKV VSQLVDANGV LKHSIKLDVI GGDGDDVVLA NASRIHYDGG AGTNTVSYAA
181 LGRQDSITVS ADGERFNVRK QLNNANVYRE GVATQTTAYG KRTENVQYRH VELARVGQVV
241 EVDTLEHVQH IIGGAGNDSI TGNAHDNFLA GGSGDDRLDG GAGNDTLVGG EGQNTVIGGA
301 GDDVFLQDLG VWSNQLDGGA GVDTVKYNVH QPSEERLERM GDTGIHADLQ KGTVEKWPAL
361 NLFSVDHVKN IENLHGSRLN DRIAGDDQDN ELWGHDGNDT IRGRGGDDIL RGGLGLDTLY
421 GEDGNDIFLQ DDETVSDDID GGAGLDTVDY SAMIHPGRIV APHEYGFGIE ADLSREWVRK
481 ASALGVDYYD NVRNVENVIG TSMKDVLIGD AQANTLMGQG GDDTVRGGDG DDVLLGDEGS
541 DLLSGDAGND DLFGGQGDDT YLFGVGYGHD TIYESGGGHD TIRINAGADQ LWFARQGNDL
601 EIRILGTDDA LTVHDWYRDA DHRVEIIHAA NQAVDQAGIE KLVEAMAQYP DPGAAAAAPP
661 AARVPDTLM QSLAVNWR

In another embodiment, the heterologous polypeptide in the chimeric protein is an antigen.

In a third aspect, the invention relates to a polynucleotide encoding the polypeptide antigen of the present invention or a chimeric protein of the present invention.

In one specific embodiment, the polynucleotide shown as SEQ ID NO. 10 encodes the chimeric protein shown as SEQ ID NO. 6, and such polynucleotide is suitable for production and purification of the polypeptide antigen shown as SEQ ID NO. 2 (RTXm).
SEQ ID NO. 10 - nucleotide sequence encoding the GST-TEV-blockII-III/V protein useful in production of the RTXm polypeptide antigen of the present invention

1 ATGTCCCCTA TACTAGGTTA TTGGAAAATT AAGGGCCTTG TGCAACCCAC TCGACTTCTT
61 TTGGAATATC TTGAAGAAAA ATATGAAGAG CATTTGTATG AGCGCGATGA AGGTGATAAA
121 TGGCGAAACA AAAAGTTTGA ATTGGGTTTG GAGTTTCCCA ATCTTCCTTA TTATATTGAT
181 GGTGATGTTA AATTAACACA GTCTATGGCC ATCATACGTT ATATAGCTGA CAAGCACAAC
241 ATGTTGGGTG GTTGTCCAAA AGAGCGTGCA GAGATTTCAA TGCTTGAAGG AGCGGTTTTG
301 GATATTAGAT ACGGTGTTTC GAGAATTGCA TATAGTAAAG ACTTTGAAAC TCTCAAAGTT
361 GATTTTCTTA GCAAGCTACC TGAAATGCTG AAAATGTTCG AAGATCGTTT ATGTCATAAA
421 ACATATTTAA ATGGTGATCA TGTAACCCAT CCTGACTTCA TGTTGTATGA CGCTCTTGAT

481 GTTGTTTTAT ACATGGACCC AATGTGCCTG GATGCGTTCC CAAAATTAGT TTGTTTTAAA
541 AAACGTATTG AAGCTATCCC ACAAATTGAT AAGTACTTGA AATCCAGCAA GTATATAGCA
601 TGGCCTTTGC AGGGCTGGCA AGCCACGTTT GGTGGTGGCG ACCATCCTCC AAAATCGGAT
661 GGTTCAACTA GTGGTTCTGG TCATCACCAT CACCATCACT CCGCGGGTCT GGTGCCACGC
721 GGTAGTACTG CAATTGGTAT GAAAGAAACC GCTGCTGCTA AATTCGAACG CCAGCACATG
781 GACAGCCCAG ATCTGGGTAC CGGTGGTGGC TCCGGTATTG AGGGACGCGG GTCCATGGAA
841 AACCTGTACT TCCAGGGCAA TATCGAGAAT CTGCACGGCT CCCGCCTAAA CGACCGCATC
901 GCCGGCGACG ACCAGGACAA CGAGCTCTGG GGCCACGATG GCAACGACAC GATACGCGGC
961 CGGGGCGGCG ACGACATCCT GCGCGGCGGC CTGGGCCTGG ACACGCTGTA TGGCGAGGAC
1021 GGCAACGACA TCTTCCTGCA GGACGACGAG ACCGTCAGCG ATGACATCGA CGGCGGCGCG
1081 GGGCTGGACA CCGTCGACTA CTCCGCCATG ATCCATCCAG GCAGGATCGT TGCGCCGCAT
1141 GAATACGGCT TCGGGATCGA GGCGGACCTG TCCAGGGAAT GGGTGCGCAA GGCGTCCGCG
1201 CTGGGCGTGG ACTATTACGA TAATGTCCGC AATGTCGAAA ACGTCATCGG TACGAGCATG
1261 AAGGATGTGC TCATCGGCGA CGCGCAAGCC AATACCCTGA TGGGCCAGGG CGGCGACGAT
1321 ACCGTGCGCG GCGGCGACGG CGATGACGTC CTGCTGGGCG ACGAGGGCTC GGACCTGCTC
1381 AGCGGCGATG CGGGCAACGA CGATCTGTTC GGCGGGCAGG GCGATGATAC TTATCTGTTC
1441 GGGGTCGGGT ACGGGCACGA CACGATCTAC GAATCGGGCG GCGGCCATGA CACCATCCGC
1501 ATCAACGCGG GGGCGGACCA GCTGTGGTTC GCGCGCCAGG GCAACGACCT GGAGATCCGC
1561 ATTCTCGGCA CCGACGATGC ACTTACCGTG CACGACTGGT ATCGCGACGC CGATCACCGG
1621 GTGGAAATCA TCCATGCCGC CAACCAGGCG GTAGACCAGG CAGGCATCGA AAAGCTGGTC
1681 GAGGCAATGG CGCAGTATCC GGACCCCTGA

In one specific embodiment, the polynucleotide shown as SEQ ID NO. 11 encodes the chimeric protein shown as SEQ ID NO. 7, and such polynucleotide is suitable for production and purification of the polypeptide antigen shown as SEQ ID NO. 3.
SEQ ID NO. 11 - nucleotide sequence encoding the 6xHis-RTX1008 protein protein useful in production of the RTX1008 polypeptide antigen of the present invention

1 ATGCATCATC ATCATCATCA TGAGCATGTG CAGCACATCA TCGGCGGGGC CGGCAACGAT
61 TCGATCACCG GCAATGCGCA CGACAACTTC CTAGCCGGCG GGTCGGGCGA CGACAGGCTG
121 GATGGCGGCG CCGGCAACGA CACCCTGGTT GGCGGCGAGG GCCAAAACAC GGTCATCGGC
181 GGCGCCGGCG ACGACGTATT CCTGCAGGAC CTGGGGGTAT GGAGCAACCA GCTCGATGGC
241 GGCGCGGGCG TCGATACCGT GAAGTACAAC GTGCACCAGC CTTCCGAGGA GCGCCTCGAA
301 CGCATGGGCG ACACGGGCAT CCATGCCGAT CTTCAAAAGG GCACGGTCGA GAAGTGGCCG
361 GCCCTGAACC TGTTCAGCGT CGACCATGTC AAGAATATCG AGAATCTGCA CGGCTCCCGC
421 CTAAACGACC GCATCGCCGG CGACGACCAG GACAACGAGC TCTGGGGCCA CGATGGCAAC
481 GACACGATAC GCGGCCGGGG CGGCGACGAC ATCCTGCGCG GCGGCCTGGG CCTGGACACG
541 CTGTATGGCG AGGACGGCAA CGACATCTTC CTGCAGGACG ACGAGACCGT CAGCGATGAC
601 ATCGACGGCG GCGCGGGGCT GGACACCGTC GACTACTCCG CCATGATCCA TCCAGGCAGG
661 ATCGTTGCGC CGCATGAATA CGGCTTCGGG ATCGAGGCGG ACCTGTCCAG GGAATGGGTG
721 CGCAAGGCGT CCGCGCTGGG CGTGGACTAT TACGATAATG TCCGCAATGT CGAAAACGTC
781 ATCGGTACGA GCATGAAGGA TGTGCTCATC GGCGACGCGC AAGCCAATAC CCTGATGGGC
841 CAGGGCGGCG ACGATACCGT GCGCGGCGGC GACGGCGATG ACGTCCTGCT GGGCGACGAG
901 GGCTCGGACC TGCTCAGCGG CGATGCGGGC AACGACGATC TGTTCGGCGG GCAGGGCGAT
961 GATACTTATC TGTTCGGGGT CGGGTACGGG CACGACACGA TCTACGAATC GGGCGGCGGC
1021 CATGACACCA TCCGCATCAA CGCGGGGGCG GACCAGCTGT GGTTCGCGCG CCAGGGCAAC
1081 GACCTGGAGA TCCGCATTCT CGGCACCGAC GATGCACTTA CCGTGCACGA CTGGTATCGC
1141 GACGCCGATC ACCGGGTGGA AATCATCCAT GCCGCCAACC AGGCGGTAGA CCAGGCAGGC
1201 ATCGAAAAGC TGGTCGAGGC AATGGCGCAG TATCCGGACC CCGGCGCGGC GGCGGCTGCC
1261 CCGCCGGCGG CGCGCGTGCC GGACACGCTG ATGCAGTCCC TGGCTGTCAA CTGGCGCTGA

In one specific embodiment, the polynucleotide shown as SEQ ID NO. 12 encodes the chimeric protein shown as SEQ ID NO. 8, and such polynucleotide is suitable for production and purification of the polypeptide antigen shown as SEQ ID NO. 4.
SEQ ID NO. 12 - nucleotide sequence encoding the 6xHis-RTX908 protein protein useful in production of the RTX908 polypeptide antigen of the present invention

1       ATGCATCATC ATCATCATCA TAAACTGGAT GTGATCGGCG GAGATGGCGA TGACGTCGTG
61      CTTGCCAATG CTTCGCGCAT CCATTATGAC GGCGGCGCGG GCACCAACAC GGTCAGCTAT
121     GCCGCCCTGG GTCGACAGGA TTCCATTACC GTGTCCGCCG ACGGGGAACG TTTCAACGTG
181     CGCAAGCAGT TGAACAACGC CAACGTGTAT CGCGAAGGCG TGGCTACCCA GACAACCGCC
241     TACGGCAAGC GCACGGAGAA TGTCCAATAC CGCCATGTCG AGCTGGCCCG TGTCGGGCAA
301     GTGGTGGAGG TCGACACGCT CGAGCATGTG CAGCACATCA TCGGCGGGGC CGGCAACGAT
361     TCGATCACCG GCAATGCGCA CGACAACTTC CTAGCCGGCG GGTCGGGCGA CGACAGGCTG
421     GATGGCGGCG CCGGCAACGA CACCCTGGTT GGCGGCGAGG GCCAAAACAC GGTCATCGGC
481     GGCGCCGGCG ACGACGTATT CCTGCAGGAC CTGGGGGTAT GGAGCAACCA GCTCGATGGC
541     GGCGCGGGCG TCGATACCGT GAAGTACAAC GTGCACCAGC CTTCCGAGGA GCGCCTCGAA
601     CGCATGGGCG ACACGGGCAT CCATGCCGAT CTTCAAAAGG GCACGGTCGA GAAGTGGCCG
661     GCCCTGAACC TGTTCAGCGT CGACCATGTC AAGAATATCG AGAATCTGCA CGGCTCCCGC
721     CTAAACGACC GCATCGCCGG CGACGACCAG GACAACGAGC TCTGGGGCCA CGATGGCAAC
781     GACACGATAC GCGGCCGGGG CGGCGACGAC ATCCTGCGCG GCGGCCTGGG CCTGGACACG
841     CTGTATGGCG AGGACGGCAA CGACATCTTC CTGCAGGACG ACGAGACCGT CAGCGATGAC
901     ATCGACGGCG GCGCGGGGCT GGACACCGTC GACTACTCCG CCATGATCCA TCCAGGCAGG
961     ATCGTTGCGC CGCATGAATA CGGCTTCGGG ATCGAGGCGG ACCTGTCCAG GGAATGGGTG
1021    CGCAAGGCGT CCGCGCTGGG CGTGGACTAT TACGATAATG TCCGCAATGT CGAAAACGTC
1081    ATCGGTACGA GCATGAAGGA TGTGCTCATC GGCGACGCGC AAGCCAATAC CCTGATGGGC
1141    CAGGGCGGCG ACGATACCGT GCGCGGCGGC GACGGCGATG ACGTCCTGCT GGGCGACGAG
1201    GGCTCGGACC TGCTCAGCGG CGATGCGGGC AACGACGATC TGTTCGGCGG GCAGGGCGAT
1261    GATACTTATC TGTTCGGGGT CGGGTACGGG CACGACACGA TCTACGAATC GGGCGGCGGC
1321    CATGACACCA TCCGCATCAA CGCGGGGGCG GACCAGCTGT GGTTCGCGCG CCAGGGCAAC
1381    GACCTGGAGA TCCGCATTCT CGGCACCGAC GATGCACTTA CCGTGCACGA CTGGTATCGC
1441    GACGCCGATC ACCGGGTGGA AATCATCCAT GCCGCCAACC AGGCGGTAGA CCAGGCAGGC
1501    ATCGAAAAGC TGGTCGAGGC AATGGCGCAG TATCCGGACC CCGGCGCGGC GGCGGCTGCC
1561    CCGCCGGCGG CGCGCGTGCC GGACACGCTG ATGCAGTCCC TGGCTGTCAA CTGGCGCTGA

In one specific embodiment, the polynucleotide shown as SEQ ID NO. 13 encodes the chimeric protein shown as SEQ ID NO. 8, and such polynucleotide is suitable for production and purification of the polypeptide antigen shown as SEQ ID NO. 4.
SEQ ID NO. 13 - nucleotide sequence encoding the 6xHis-RTX770 protein protein useful in production of the RTX770 polypeptide antigen of the present invention

1 ATGCATCATC ATCATCATCA TAGCAGCGTG ATCGGGGTGC AGACGACAGA GATCTCCAAG
61 TCGGCGCTCG AACTGGCCGC CATTACCGGC AACGCGGACA ACCTGAAATC CGTCGACGTG
121 TTCGTGGACC GCTTCGTCCA GGGCGAGCGG GTGGCCGGCC AGCCGGTGGT CCTCGACGTC
181 GCCGCCGGCG GCATCGATAT CGCCAGCCGC AAGGGCGAGC GGCCGGCGCT GACGTTCATC
241 ACGCCGCTGG CCGCGCCAGG AGAAGAGCAG CGCCGGCGCA CGAAAACGGG CAAGAGCGAA
301 TTCACCACAT TCGTCGAGAT CGTGGGCAAG CAGGACCGCT GGCGCATCCG GGACGGCGCG
361 GCCGACACCA CCATCGATCT GGCCAAGGTG GTGTCGCAAC TGGTCGACGC CAATGGCGTG
421 CTCAAGCACA GCATCAAACT GGATGTGATC GGCGGAGATG GCGATGACGT CGTGCTTGCC
481 AATGCTTCGC GCATCCATTA TGACGGCGGC GCGGGCACCA ACACGGTCAG CTATGCCGCC
541 CTGGGTCGAC AGGATTCCAT TACCGTGTCC GCCGACGGGG AACGTTTCAA CGTGCGCAAG

601 CAGTTGAACA ACGCCAACGT GTATCGCGAA GGCGTGGCTA CCCAGACAAC CGCCTACGGC
661 AAGCGCACGG AGAATGTCCA ATACCGCCAT GTCGAGCTGG CCCGTGTCGG GCAAGTGGTG
721 GAGGTCGACA CGCTCGAGCA TGTGCAGCAC ATCATCGGCG GGGCCGGCAA CGATTCGATC
781 ACCGGCAATG CGCACGACAA CTTCCTAGCC GGCGGGTCGG GCGACGACAG GCTGGATGGC
841 GGCGCCGGCA ACGACACCCT GGTTGGCGGC GAGGGCCAAA ACACGGTCAT CGGCGGCGCC
901 GGCGACGACG TATTCCTGCA GGACCTGGGG GTATGGAGCA ACCAGCTCGA TGGCGGCGCG
961 GGCGTCGATA CCGTGAAGTA CAACGTGCAC CAGCCTTCCG AGGAGCGCCT CGAACGCATG
1021 GGCGACACGG GCATCCATGC CGATCTTCAA AAGGGCACGG TCGAGAAGTG GCCGGCCCTG
1081 AACCTGTTCA GCGTCGACCA TGTCAAGAAT ATCGAGAATC TGCACGGCTC CCGCCTAAAC
1141 GACCGCATCG CCGGCGACGA CCAGGACAAC GAGCTCTGGG GCCACGATGG CAACGACACG
1201 ATACGCGGCC GGGGCGGCGA CGACATCCTG CGCGGCGGCC TGGGCCTGGA CACGCTGTAT
1261 GGCGAGGACG GCAACGACAT CTTCCTGCAG GACGACGAGA CCGTCAGCGA TGACATCGAC
1321 GGCGGCGCGG GGCTGGACAC CGTCGACTAC TCCGCCATGA TCCATCCAGG CAGGATCGTT
1381 GCGCCGCATG AATACGGCTT CGGGATCGAG GCGGACCTGT CCAGGGAATG GGTGCGCAAG
1441 GCGTCCGCGC TGGGCGTGGA CTATTACGAT AATGTCCGCA ATGTCGAAAA CGTCATCGGT
1501 ACGAGCATGA AGGATGTGCT CATCGGCGAC GCGCAAGCCA ATACCCTGAT GGGCCAGGGC
1561 GGCGACGATA CCGTGCGCGG CGGCGACGGC GATGACGTCC TGCTGGGCGA CGAGGGCTCG
1621 GACCTGCTCA GCGGCGATGC GGGCAACGAC GATCTGTTCG GCGGGCAGGG CGATGATACT
1681 TATCTGTTCG GGGTCGGGTA CGGGCACGAC ACGATCTACG AATCGGGCGG CGGCCATGAC
1741 ACCATCCGCA TCAACGCGGG GGCGGACCAG CTGTGGTTCG CGCGCCAGGG CAACGACCTG
1801 GAGATCCGCA TTCTCGGCAC CGACGATGCA CTTACCGTGC ACGACTGGTA TCGCGACGCC
1861 GATCACCGGG TGGAAATCAT CCATGCCGCC AACCAGGCGG TAGACCAGGC AGGCATCGAA
1921 AAGCTGGTCG AGGCAATGGC GCAGTATCCG GACCCCGGCG CGGCGGCGGC TGCCCCGCCG
1981 GCGGCGCGCG TGCCGGACAC GCTGATGCAG TCCCTGGCTG TCAACTGGCG CTGA

In a fourth aspect, the present invention relates to a vector comprising the polynucleotide as described above (SEQ ID NO. 10, 11, 12 and 13). Preferably, the plasmid vector further comprises an origin of replication, such as the high copy replication origin colE1, a marker gene for selection for the presence of the vector in a cell, such as an antibiotic resistance gene, a T7 phage terminator of transcription, a 5' leader region and translation initiation site from gene 10 of phage T7, a gene for a transcriptional repressor, such as the LacI repressor and a lac operator site binding LacI, enabling inducible and thus regulated expression of the polynucleotides (genes) encoding the polypeptide antigen of the present invention or a chimeric protein of the present invention, such as through transcription by the T7 RNA polymerase or the *E. coli* RNA polymerase under the control of a LacI-regulated T7 or Plac promoter, respectively.

In a fifth aspect, the present invention relates to a host cell genetically engineered with the polynucleotide or with the vector described previously, such as the *Escherichia coli* B strain BL21/pMM100lacI^{q} expressing the LacI repressor form the pMM100 lacI^{q} plasmid and in which high level production of the RTX1008, RTX908 or RTX770 is achieved, respectively, or the *Escherichia coli* B strain expressing the T7 RNA polymerase under lacUV5 promoter control, such as *Escherichia coli* BL21λDE3 or the *Escherichia coli* BL21(DE3)pLysS, in which high level production of the RTXm polypeptide antigen of the present invention or a chimeric protein of the present invention is achieved by induction in the presence of an inducer compound, such as isopropyl β-D-1-thiogalactopyranoside. An alternative host cell may be a recombinant *Bordetella* cell genetically engineered with the polynucleotide or with the vector described previously to produce and secrete through the Type I secretion system the polypeptide antigen of the present invention, such as RTXm, RTX1008, RTX908 or RTX770, or the chimeric protein of the present invention.

Preferably, the host cell is further genetically engineered with a polynucleotide or with a vector to express the CyaA toxin-acylating enzyme CyaC from a gene cassette described previously (Osicka et al. 2000, Infect. Immun. 68, pp. p. 247-256; Holubova et al. 2012, Infect Immun. 80, pp.1181-1192). This is particularly preferred when the sequence of the antigen of the present invention contains the lysine K860 and/or K983 residues, the acylation of which is essential for strong binding into cell membrane through interaction with the receptor CD11b/CD18 (Masin et al. 2005, Biochemistry 44, pp. 12759-12766).

In a sixth aspect, the polypeptide antigens and chimeric proteins according to the invention are suitable for use in prophylactic treatment and especially in vaccination and/or in therapy, including in immunotherapy, in particular for eliciting an immune response in a subject, preferably a human subject.

In a seventh aspect, the present invention relates to a vaccine against a disease caused by a bordetella pathogen, preferably selected from *Bordetella pertussis, Bordetella parapertussis* and *Bordetella bronchiseptica,* in particular against pertussis or whooping cough, comprising the recombinant polypeptide antigen derived from CyaA according to the present invention or the chimeric protein of the present invention, at least one pharmaceutically acceptable auxiliary substance, and optionally an adjuvant. In some embodiments, the vaccine is selected from a vaccine for paedriatric use, a vaccine for maternal immunization and/or a booster vaccine. The vaccine may be for human use (human vaccine), or for veterinary use (veterinary vaccine). In some embodiments, the vaccine is a combination vaccine, such as a pentavaccine or a hexavaccine against five or six infectious diseases, respectively.

In a particularly preferred embodiment, the vaccine comprises the polypeptide antigen of the present invention or a chimeric protein according to the present invention, and an aluminium adjuvant such as aluminium hydroxide or aluminium phosphate or aluminium gel or alum. In some embodiments, the polypeptide antigen is selected from RTX1008, RTX908 and RTX770. In some embodiments, the aluminium adjuvant is aluminium hydroxide, including hydrated aluminium hydroxide, or alum.

The auxiliary substances include pharmaceutically acceptable solvents, such as water, alcohols, lactose, surfactants and emulsifiers, such as polysorbates (e.g. Tween 80), pH-adjusting components, inorganic salts, adjuvants, etc.

Examples of suitable adjuvants comprise aluminium salts, such as hydrated aluminium hydroxide or aluminium phosphate, alum, aluminium gel, lipopolysaccharide derived monophosphoryl lipid A, squalene-based MF59 adjuvant, chitosan etc. In some embodiments, the aluminium adjuvant is aluminium hydroxide.

The vaccine may preferably be in the form of suspension, more preferably aqueous suspension. The routes of administration may include injection, such as intramuscular injection, subcutaneous injection, intradermal injection and sublingual or intranasal administration.

The present invention further provides the polypeptide antigen according to the present invention or the chimeric protein according to the present invention for use in a method of treatment and/or prevention of a disease caused by a bordetella pathogen, preferably selected from *Bordetella pertussis, Bordetella parapertussis* and *Bordetella bronchiseptica,* in particular against pertussis or whooping cough disease.

The present invention further provides a method of treatment and/or prevention of a disease caused by a bordetella pathogen, preferably selected from *Bordetella pertussis, Bordetella parapertussis* and *Bordetella bronchiseptica,* in particular against pertussis or whooping cough, comprising the step of administering at least one polypeptide antigen according to the present invention or at least one chimeric protein according to the present invention to a subject in need of such treatment and/or prevention.

### Brief description of the drawings

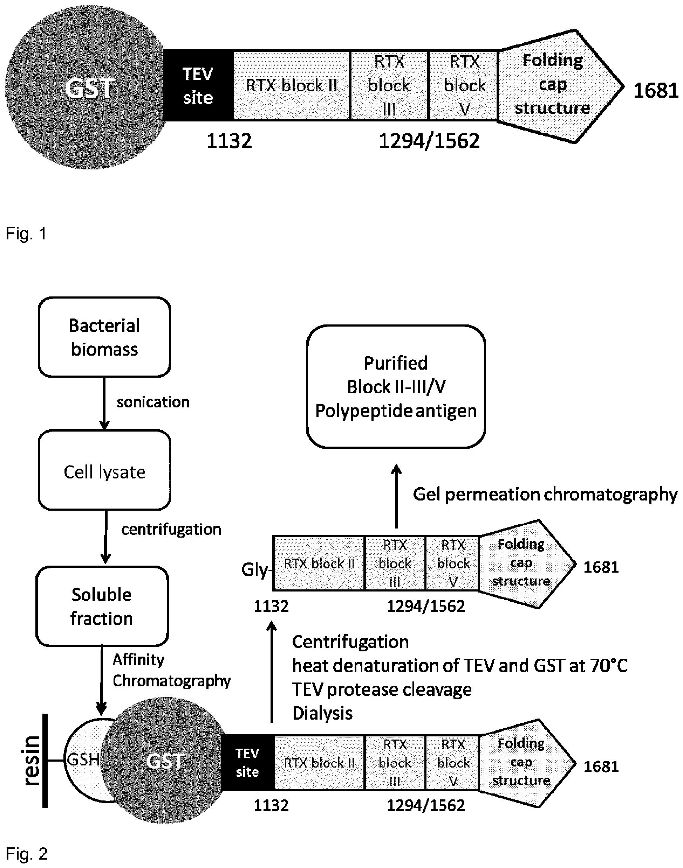

**Figure 1** depicts the scheme of the construct used in production of the recombinant RTXm polypeptide antigen of the present invention. The polypeptide consists of an N-terminal GST tag, bearing a TEV protease cleavage site of the sequence ENLYFQG on its C-terminal end and fused by recombinant DNA technology to the segment comprising residues 1132 to 1294 of RTX block Blocks II and III, which has been fused to residues 1562 to 1681, comprising a fragment of RTX repeat block V and the C-terminal calcium-dependent folding-initiating and scaffolding structure of CyaA (Bumba et al. 2016, Mol. Cell 62, pp. 47-62).
**Figure 2** documents the scheme of affinity purification of the fusion polypeptide of the present invention on glutathione agarose beads, followed by dialysis, cleavage with the TEV protease, heat denaturation of contaminating proteins and of the TEV protease, separation of the precipitate by centrifugation and separation of the GST tag moiety from the RTXm polypeptide antigen of the present invention by gel permeation chromatography.
**Figure 3** documents the calcium-dependent *in vitro* folding of the purified BlockII-III/V antigen RTXm polypeptide of the present invention as followed by CD spectroscopy. (A) CD spectra of the RTXm polypeptide antigen at increasing calcium ion concentrations. (B) Calcium titration curve of the RTXm polypeptide antigen.

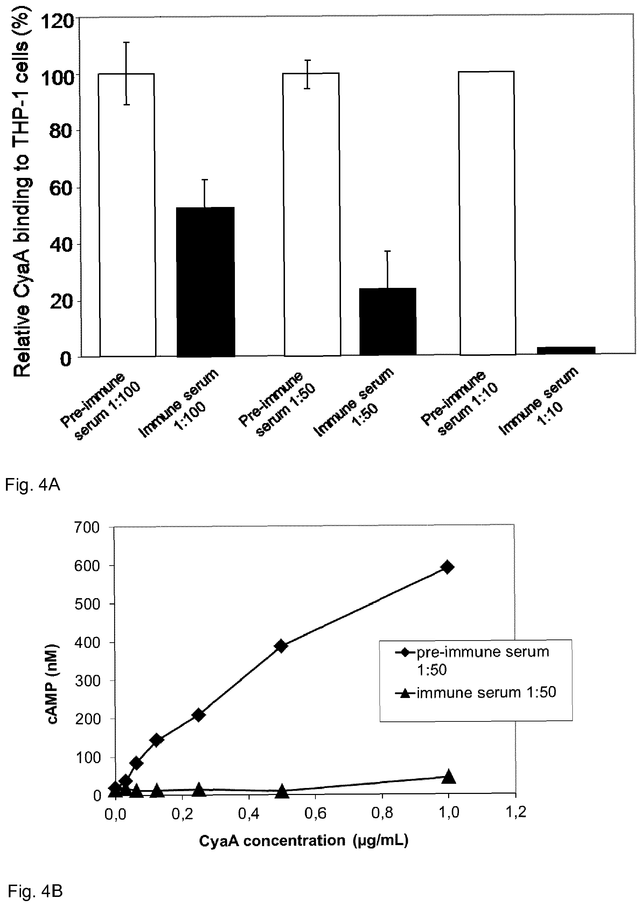

**Figure 4** shows the toxin-neutralizing activity of mouse sera raised by intraperitoneal immunization of mice by two injections in two weeks interval of 30 µg of purified RTXm polypeptide antigen of the present invention in incomplete Freund's adjuvant.
   (**A**) inhibition of CyaA toxin binding to human THP-1 macrophage cells, expressing the CR3 receptor of the toxin, in the presence of immune or pre-immune mouse sera diluted 1:100, 1:50 and 1:10, respectively.
   (**B**) inhibition of CyaA-catalyzed cAMP formation in human THP-1 macrophage cells in the presence of 1:50 diluted pre-immune or immune sera raised in mice by vaccination with the purified RTXm polypeptide antigen of the present invention.

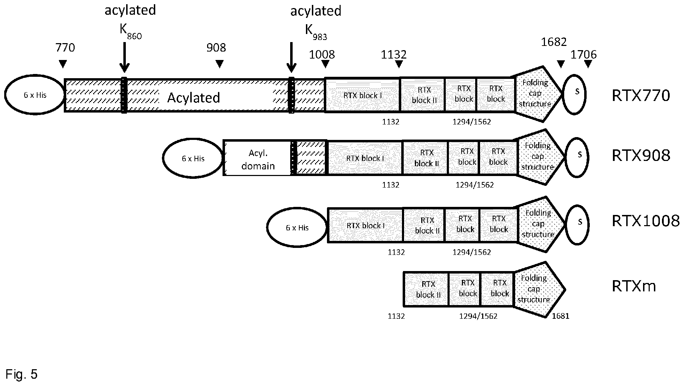

**Figure 5** depicts the schemes of the constructs used in production of the extended recombinant RTXm and RTXm-derived RTX770, RTX908 and RTX1008 polypeptide antigens of the present invention. The polypeptides all comprise the segment consisting of residues 1132 to 1294 of RTX repeat blocks II and III, which has been fused to residues 1562 to 1681, comprising a fragment of RTX repeat block V and the C-terminal calcium-dependent folding-initiating and scaffolding structure of CyaA (Bumba et al. 2016, Mol. Cell 62, pp. 47-62). The RTX770, RTX908 and RTX1008 antigen polypeptides are then extended at their C-terminal ends by the segment of the Type I secretion system signal (Sebo and Ladant 1993, Mol. Microbiol. 1993, 9, pp. 999-1009) up to residue 1706. The N-terminal ends of the RTX770, RTX908 and RTX1008 antigens are extended up to the residues 770, 908 and 1008, respectively, which are fused to a C-terminal 6 x His affinity purification tag. The RTX770 and RTX908 antigens harbor the two or a single acylation site which are lysine residues K860 and K983 indicated by arrows. These antigens were produced in the presence of the toxin acyl-transferase enzyme CyaC in *E. coli* cells and fatty-acylation of both the K860 and K983 residues in purified RTX770 and of the K983 residue only in RTX908, respectively, was confirmed by LC-FT MS/MS analysis.

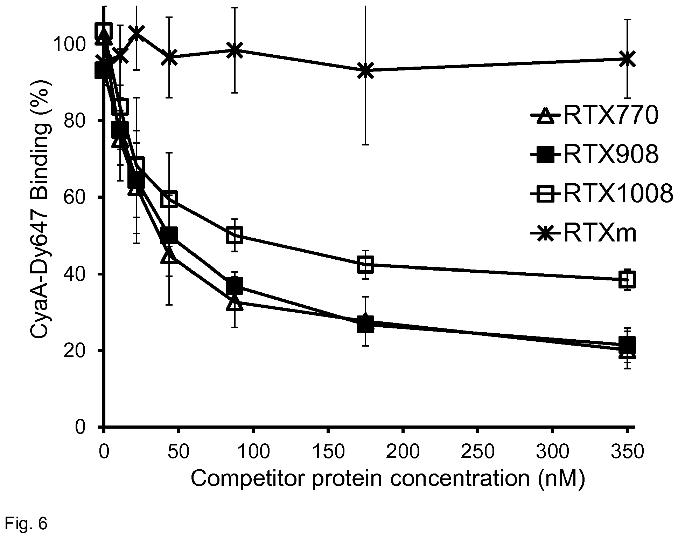

**Figure** 6 depicts the comparison of the capacity of the purified RTXm, RTX770, RTX908 and RTX1008 antigens to engage the CD11b/CD18 receptor (CR3) expressed on transfected CHO cells (Osicka et al, eLife, 2015) and competitively inhibit the binding of the fluorescently labeled full-length CyaA-AC⁻-Dy647 toxoid. Aliquots of 100,000 CHO cells expressing the CR3 receptor (Osicka et al, eLife, 2015) were incubated in a final volume of 0.2 ml of cHBSS buffer on ice with 5.6 nM CyaA-AC⁻-Dy647 toxoid in the presence of the tested competitor proteins at the indicated final concentrations. After 30 minutes the Hoechst 33258 vital stain was added (1 µg/ml) and the samples were immediately analyzed for binding of the fluorescently labeled CyaA-AC⁻-Dy647 toxoid by flow cytometry, using singlets gating. The mean fluorescence intensity of cell-bound CyaA-AC⁻-Dy647 toxoid in the absence of competitor protein was taken as 100%.

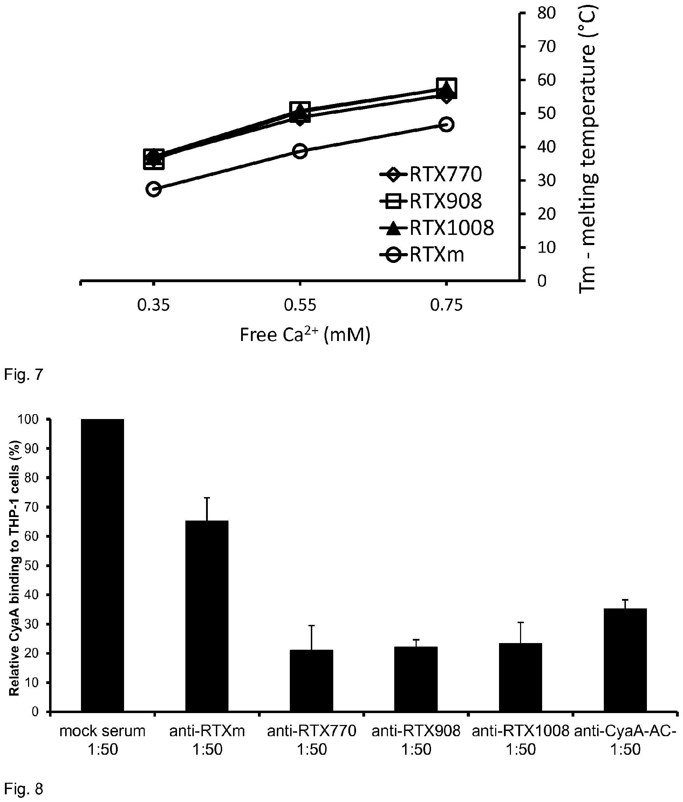

**Figure** 7 shows the comparison of thermal stability of the purified folded RTXm, RTX770, RTX908 and RTYX1008 antigens at three different concentrations of free Ca²⁺ ions. The purified antigen proteins of the present invention were dialyzed overnight at 4°C against 5 mM Tris-Cl pH 8, 50 mM NaCl, 1.5 mM CaCl₂ buffer and diluted to a concentration of 0.2 mg/ml in the same buffer. 10 µl aliquots of the individual proteins were mixed 1:1 with 10 µl aliquots of 0 mM, 0.4 mM or 0.8 mM EGTA solutions in mM Tris-Cl pH 8, 50 mM NaCl, to yield a final protein concentration of 0.1 mg/ml and adjust the final free Ca²⁺ ion concentrations to 0.35, 0.55 and 0.75 mM, respectively. 10 µl aliquots of such protein samples were loaded into capillary tubes of the Prometheus NT.48 nanoDSF instrument (NanoTemper Technologies GmbH, Munich, Germany) and unfolding temperatures of the individual proteins at indicated free Ca²⁺ ion concentrations were determined by differential scanning fluorimetry, using the PR - ThermControl v. 2.1.1. software for scanning and data analysis according to manufacturer's instructions.
**Figure** 8 shows the inhibition of binding to CD11b/CD18-expressing human THP-1 myeloid macrophage cells of the CyaA toxin that has been preincubated with 1:50 diluted polyclonal mouse sera raised by immunization with the indicated antigens as described in Example 7, namely anti-RTXm, anti-RTX770, anti-RTX908, anti-RTX1008 and anti-CyaA-AC⁻ toxoid sera, using sera from mock-vaccinated mice as negative control. THP-1 cells were cultured at 37°C in a humidified air/CO₂ atmosphere in RPMI medium supplemented with 10% heat-inactivated FCS, penicillin (100 i.u./ml), streptomycin (100 µg/ml) and amphotericin B (250 ng/ml). Prior to assays, RPMI was replaced with D-MEM medium (1.9 mM Ca²⁺) without FCS. CyaA-wt (1 µg/ml) was preincubated in D-MEM medium for 15 minutes at 4 °C in the presence of sera (1:50). After preincubation, THP-1 cells (10⁶) were added and the mixture was incubated for additional 30 min at 4°C. Unbound CyaA toxin was removed by three washes in D-MEM and after the transfer to a fresh tube, cells were lyzed with 0.1% Triton X-100 for determination of cell-bound AC enzyme activity. Binding activities are expressed as percentages of CyaA binding to THP-1 cells in the presence of mock serum (from mice that received PBS+Al(OH)₃) taken as 100%. N=4.

### Examples

### Example 1. Construction, production and purification of the polypeptide RTXm antigen

Towards generating the expression vector for production of the GST-fused polypeptide antigen of the present invention, the plasmid pET42b (Novagen)-derived expression vector, with inserted synthetic gene and encoding the open reading frame of the SEQ ID NO. 10, was constructed by *in vitro* DNA recombination techniques, yielding the pGST-TEV-BlockII-III/V vector (Fig. 1). The construction of the expression vector was verified by DNA sequencing and recombinant polypeptide antigen was expressed under T7 promoter control in *Escherichia coli* BL21(DE3) cells form the pGST-TEV-BlockII-III/V vector. Production of the GST-fused polypeptide antigen synthesis was induced in exponential 250 ml cultures grown at 37°C with shaking in Luria-Bertani broth using 1mM isopropyl 1-thio-β-D-galactopyranoside for 4 h. The scheme of purification of the polypeptide antigen of the present invention is depicted in Fig. 2. Harvested bacterial cells were disrupted by ultrasound, cell debris was separated by centrifugation at 30,000 *g* for 30 minutes at 4°C and the cytosolic extract containing the GST-TEV-BlockII-III/V fusion protein was loaded onto a Glutathione-Agarose column equilibrated with the binding buffer (50 mM Tris-HCl, pH 8.0, 150 mM NaCl, 10 mM CaCl₂). Unbound protein was removed by washing of the resin with 50 bed volumes of the binding buffer and the GST-TEV-BlockII-III/V fusion protein was next eluted with 50 mM Tris-HCl, pH 8.0, 150 mM NaCl, 10 mM CaCl₂ buffer containing 10 mM glutathione. Fractions containing the fusion protein were pooled and dialyzed overnight against 100 volumes of 20 mM Tris-HCl, pH 8.0, 150 mM NaCl buffer at 4°C. In parallel, the GST tag was cleaved-off by digesting the GST-TEV-BlockII-III/V fusion protein with added TEV protease (1:20 w/w) for 12 hours at 4°C.. The mixture was next incubated at 70°C for 30 minutes to denature and precipitate the TEV protease, the GST tag moiety and the rests of contaminating *E. coli* proteins. The precipitate was removed by centrifugation at 30,000 *g* for 15 minutes and the cleared solution was loaded onto a 10/300 Superdex 75 column equilibrated with 20 mM Tris, pH 8.0, 150 mM NaCl, 2 mM CaCl₂, on which the RTXm polypeptide antigen of the present invention was separated from contaminants in a single elution peak of the calcium loaded and folded protein.

### Example 2. Calcium-dependent folding of the BlockII-III/V polypeptide antigen RTXm

The CR3 receptor binding segment of CyaA within the RTX blocks II and III needs to fold into RTX β-roll structures in the presence of millimolar calcium ion concentrations in order to form the structure recognizing the CD11b subunit of the CD11b/CD18 heterodimeric complex (Osicka et al. 2015 eLife 4: e10766; Bumba et al. 2016, Mol. Cell 62, pp. 47-62). Therefore, the capacity of the BlockII-III/V polypeptide antigen, produced as described under Example 1, was assessed for the capacity to undergo calcium-dependent folding into the β-roll structures characteristic for the RTX blocks of CyaA. Titration of the protein with Ca²⁺ ions was followed by far-UV circular dichroism (CD) spectroscopy. Towards this aim, the BlockII-III/V polypeptide-bound calcium ions were removed by overnight dialysis against 100 volumes of 20 mM Tris, pH 8.0, 150 mM NaCl, 2 mM EDTA buffer, followed by dialysis against 100 volumes of 20 mM Tris, pH 8.0, 150 mM NaCl without EDTA. The BlockII-III/V polypeptide was titrated with calcium ions and CD spectra were acquired at each step of addition of increasing Ca²⁺ ion concentrations up to 10 mM final. The far-UV CD spectra were recorded on a Jasco-815 spectropolarimeter in rectangular quartz Suprasil cells of 1-mm path length (110-QS, Hellma). Protein samples (100 µg/ml) were diluted in 5 mM Tris-HCl (pH 7.4), 50 mM NaCl and measured for wavelengths from 195 to 280 nm at 20 °C. Two spectra accumulations with standard instrument sensitivity and scanning speed of 10 nm/min with response time of 16 ms were acquired. Calcium titration experiments were performed by successive addition of CaCl₂. The spectra of the buffers were subtracted from the protein spectra and the mean molar ellipticity (Θ) was expressed in degrees square centimeter per decimole [deg.cm-2.dmol-1]. As documented in Fig. 3A, a prominent negative peak at 217 nm, corresponding to formation of the RTX β-roll structures (Chenal et al. 2010, Biophys J. 99, pp. 3744-3753), was observed with increasing Ca²⁺ ions. Transition of the BlockII-III/V polypeptide antigen from an intrinsically disordered state into the folded β-roll state occurred as a two-stage process, triggered at -0.3 mM Ca²⁺ and completed at < 2 mM Ca²⁺ (Fig. 3B). These data demonstrate that the BlockII-III/V polypeptide antigen of the present invention responded to calcium ion binding by forming RTX β-roll structures in a very similar calcium ion concentration dependent way as the entire RTX domain polypeptide of CyaA (Bumba et al. 2016, Mol. Cell 62, pp. 47-62).

### Example 3. The purified BlockII-III/V polypeptide antigen RTXm induces CyaA-neutralizing antibodies in mice.

To determine if the calcium loaded and folded BlockII-III/V recombinant polypeptide antigen RTXm of Example 1 can present protective epitopes to B lymphocytes *in vivo* and induce formation of polyclonal CyaA-neutralizing antibodies, the BlockII-III/V polypeptide antigen RTXm of Example 1 was used for vaccination of mice. Groups of 4 Balb/c were immunized twice in two weeks interval intraperitoneally with 30 µg of BlockII-III/V polypeptide antigen RTXm in incomplete Freund's adjuvant. Two weeks after the last injection, mice were euthanized and bled to collect sera. The toxin-neutralizing activity of the antibodies in the collected sera was then assessed in experiments on human THP-1 macrophage cells that express the CR3 (CD11b/CD18 complex) receptor of CyaA on the cell surface. The target cells were grown at 37°C in a humidified air/CO₂ (19:1) atmosphere in RPMI medium supplemented with 10% (v/v) heat-inactivated fetal bovine serum, penicillin (100 i.u./ml), streptomycin (100 µg/ml) and amphotericin B (250 ng/ml). Prior to assays, RPMI was replaced with D-MEM medium (1.9 mM Ca²⁺) without FCS and the cells were allowed to rest in D-MEM for 1 h at 37°C in a humidified 5% CO₂ atmosphere (Basler et al. 2006 Infect Immun 74, pp. 2207-2214). Suspensions of THP-1 cells (10⁶/ml) in D-MEM medium containing different dilutions of pre-immune or immune sera (1:10, 1:50, 1:100) of mice were next prepared. CyaA toxin that has been preincubated for 30 minutes on ice with different dilutions of pre-immune or immune mouse serum and added to THP-1 cells to reach a final concentration of the toxin of 1 µg/ml. After co-incubation of cells with toxin in the presence of sera for 30 minutes on ice, the THP-1 cells were washed repeatedly with cold D-MEM medium and the amount of cells associated (CR3 receptor-bound) CyaA toxin was determined by measuring the amount of adenylyl cyclase (AC) enzyme activity of CyaA present in cell lyzates. The AC enzyme activities were measured in the presence of 1 µM calmodulin as previously described (Ladant et al. 1988, J Biol Chem. 263, pp. 2612-2618. One unit of AC activity corresponds to 1 µmol of cAMP formed per min at 30°C, pH 8.0.

As documented in Fig. 4A, preincubation of CyaA with 1:100 diluted immune sera raised in mice against the polypeptide antigen BlockII-III/V and presence of the 1:100 diluted immune sera during co-incubation of such-treated CyaA with THP-1 caused 50% decrease of the amount of cell-bound CyaA, as compared to pre-incubation and presence of equally (1:100) diluted mouse pre-immune sera. Preincubation of CyaA with 1:50 and 1:10 diluted immune sera resulted in further decrease of the amount of cell-bound CyaA to 20% and about 1%, respectively, as compared to preincubation with correspondingly diluted mouse pre-immune sera. This clearly shows that immunization of mice with the calcium-loaded and thus folded BlockII-III/V polypeptide antigen in incomplete Freund's adjuvant induces production of antibodies that recognize the intact CyaA toxin molecule and block its capacity to interact with THP-1 cells through the CR3 receptor. This is most likely due to induction of antibodies that recognize the conformational protective epitopes formed within the RTX blocks II and III that comprise the toxin site involved in binding to the CR3 receptor. In contrast, immunization with unfolded BlockII-III/V polypeptide antigen formulated with calcium-ion sequestering aluminium hydroxide adjuvant failed to induce CyaA binding neutralizing antibody responses in mice and constructs differing from the BlockII-III/V polypeptide antigen in the site of fusion of the block III with fragment of the block V, which were unable to undergo the calcium-dependent folding due to disruption of the β-roll structure at the junction of RTX blocks III and V, were as well unable to induce CyaA-neutralizing antibody responses in immunized mice, even if administered in incomplete Freund's adjuvant.
To corroborate the above observation the capacity of immune sera raised against calcium-loaded and folded BlockII-III/V polypeptide antigen of Example 1 was assessed in an assay for inhibition of the biological activity of CyaA that consists in its CR3 receptor-dependent penetration into cytosol of myeloid phagocytic cells, such as the THP-1 cells and catalytic conversion of cytosolic ATP to the cAMP second messenger molecule, which at increased concentrations dysregulates cellular signaling and ablates the bactericidal capacities of myeloid phagocytic cells (Confer D. L. and Eaton J.W. 1982, Science 217, pp. 948-950; Kamanova et al. 2008, J. Immunol. 181, pp. 5587-5597; Cerny et al. 2017, J. Immunol. 198, pp. 1285-1296). CyaA toxin at concentrations of 30 to 1000 ng/mL in D-MEM was preincubated for 30 minutes at room temperature with 1:50 diluted pre-immune or immune serum and mixed with 2 x 10⁵/ml THP-1 cells. Incubation in D-MEM medium containing 1:50 diluted pre-immune or immune sera was continued for 30 minutes at 37°C. The reactions were stopped by 0.2% Tween-20 in 100 mM HCl. The samples were boiled for 15 min at 100°C, neutralized by addition of 150 mM unbuffered imidazole and cAMP was measured by a competitive immunoassay (Masin et al. 2016 Sci. Rep. 6(1):29137 doi: 10.1038/srep29137). As documented in Fig. 4B, exposure of CyaA to 1:50 diluted immune sera raised against the BlockII-III/V polypeptide antigen reduced by over an order of magnitude (>90%) the capacity of CyaA to penetrate THP-1 cells and elevate the cytosolic cAMP concentrations (Fig. 4B). The immune serum, hence, contained CyaA toxin-neutralizing antibodies induced by the conformational protective epitopes formed upon calcium-dependent folding of the BlockII-III/V polypeptide antigen.

### Example 4. Construction, production and purification of the extended BlockII-III/V-derived polypeptide antigens RTX770, RTX908 and RTX1008

Towards generating the expression vectors for production of the RTX770, RTX908 and RTX1008 antigens of the present invention, the plasmid pT7CT7ACT/E5-mutNdeI (Holubova et al. 2012, Infect Immun. 80, pp.1181-1192), carrying the *cyaC* gene expression cassette was used. The respective inserted synthetic genes encoding the open reading frames of the above described sequences, were inserted to replace the *cyaA* gene open reading frame in pT7CT7ACT1/E5-mutNdeI by *in vitro* DNA recombination techniques, yielding the pT7CT7-RTX770, pT7CT7-RTX908 and pT7CT7-RTX1008 vectors, respectively. The construction of the expression vectors was verified by DNA sequencing. The recombinant and CyaC-acylated polypeptide antigens RTX770 and RTX908 and the non-acylated RTX1008 antigen were expressed under Plac promoter control in *Escherichia coli* BL21/pMM1001ac^{Iq} cells from the pT7CT7-RTX770, pT7CT7-RTX908 and pT7CT7-RTX1008 vectors, respectively. Synthesis of the RTX770, RTX908 and RTX1008 polypeptide antigens was induced in exponential 500 ml cultures grown at 37°C with shaking in Luria-Bertani broth using 1 mM isopropyl 1-thio-β-D-galactopyranoside for 4 h. Harvested bacterial cells were disrupted by ultrasound, inclusion bodies of the individual antigen proteins were separated by centrifugation at 30,000 *g* for 30 minutes at 4°C, washed in 50 mM Tris-HCl, pH 8.0, 120 mM NaCl and extracted with 8 M urea in 50 mM Tris-HCl, pH 8.0, 120 mM NaCl (UTN). The individual urea extracts containing the RTX770, RTX908 and RTX1008 polypeptide antigens, respectively, were loaded onto DEAE-Sepharose columns equilibrated with the binding buffer UTN. Unbound proteins were removed by washing of the resin beds in columns with 20 bed volumes of the binding buffer, followed by removal of endotoxin by washing resin beds in columns by 20 bed volumes of 6 Urea in 50 mM Tris-HCl, pH 8.0, 120 mM NaCl and 1 % Triton X-100. The resin beds in columns were next washed by 10 bed volumes of the binding buffer UTN and the RTX770, RTX908 and RTX1008 antigens were individually eluted with 8 Urea in 50 mM Tris-HCl, pH 8.0, 250 mM NaCl. Fractions of each antigen were pooled, the purity and protein content of the pools were determined by Bradford assay and SDS-PAGE analysis and the pools of the RTX770, RTX908 and RTX1008 antigens were stored frozen at -20°C until use.

### Example 5. N-terminal and C-terminal extensions of the BlockII-III/V-antigen enhance the thermal stability of the calcium-induced structural fold of the RTX1008, RTX908 and RTX770 antigens.

The CR3 receptor binding segment of CyaA within the RTX blocks II and III needs to fold in the presence of millimolar calcium ion concentrations in order to form the structure recognizing the CD11b subunit of the CD11b/CD18 heterodimeric complex (Osicka et al. 2015 eLife 4: e10766; Bumba et al. 2016, Mol. Cell 62, pp. 47-62). The capacity of the RTX blocks II and III to bind the CD11b subunit then depends on the stability of the calcium-induced fold of the RTX blocks II and III, which is characterized by the temperature of thermal unfolding (Tm - melting temperature) in the presence of a defined concentration of free Ca²⁺ ions. Therefore, the Tm of the fold of the BlockII-III/V antigen, produced as described under Example 1 and the Tm values of the extended BlockII-III/V-derived polypeptide antigens RTX770, RTX908 and RTX1008, produced as described under Example 4, were assessed using nano differential scanning fluorimetry (nanoDSF). The four protein samples were dialyzed overnight at 4°C against 100 volumes of 5 mM Tris-Cl pH8, 50 mM NaCl, 1.5 mM CaCl₂ buffer and diluted to a concentration of 0.2 mg/ml. 10 µl aliquots of the individual proteins were mixed 1:1 with 10 µl aliquots of 0 mM, 0.4 mM or 0.8 mM EGTA solutions prepared in the 5 mM Tris-Cl pH8, 50 mM NaCl, so as to yield a final protein concentration of 0.1 mg/ml and to adjust by EGTA the final free Ca²⁺ ion concentrations to 0.35, 0.55 and 0.75 mM, respectively. 10 µl aliquots of such protein samples were loaded into capillary tubes of the Prometheus NT.48 nanoDSF instrument (NanoTemper Technologies GmbH, Munich, Germany) and unfolding temperatures of the individual proteins at the indicated free Ca²⁺ ion concentrations were determined by differential scanning fluorimetry, by measuring the intrinsic fluorescence of tyrosin and tryptophan residues, using the PR - ThermControl v. 2.1.1. software data acquisition and analysis according to manufacturer's instructions. Thermal unfolding of the protein in the presence of 0.35, 0.55 and 0.75 mM concentrations of free Ca²⁺ ions was followed by measurement of intrinsic fluorescence of tyrosine and tryptophan residues. As documented in Figure 7, at three different free Ca²⁺ concentrations the extended BlockII-III/V-derived RTX1008, RTX908 and RTX770 antigens exhibited Tm values higher by 10 to 12 °C than was the Tm value determined for the BlockII-III/V antigen RTXm. These data demonstrate that the stability of the calcium-induced fold of the BlockII-III/V polypeptide antigen of the present invention is enhanced upon its extension at the N-terminal to at least the residue 1008 to comprise the entire RTX block I and at C-terminal end to residue 1706 to comprise the sequence of the secretion signal.

### Example 6. N-terminal and C-terminal extensions of the BlockII-III/V-antigen enhance the CD11b/CD18-binding capacity of the antigens RTX1008, RTX908 and RTX770 and enable them to compete for binding to the CR3 receptor with the full-length CyaA-AC-toxoid.

The CyaA toxin binds the CD11b subunit of the CR3 (CD11b/CD18 complex) receptor of myeloid phagocytic cells through a segment of the RTX domain, which is roughly delimited by residues 1166 and 1287 of the RTX blocks II and III (Guermonprez, et al. 2001, J Exp Med 193, pp. 1035-1044; El Azami El Idrisi et al. 2003 JBC 278, pp. 38514-38521; Osicka et al. 2015 Elife 4: e10766). As documented in Example 2, this segment folds highly cooperatively at millimolar calcium concentrations into a Ca²⁺-loaded structure consisting of RTX β-rolls (Bumba et al. 2016, Mol. Cell 62, pp. 47-62). The affinity of this binding interaction is expected to depend on structural stability of the folded receptor-binding segment, which is shown under Example 5 to be enhanced upon N-terminal and C-terminal extension of the BlockII-III/V antigen RTXm polypeptide. Therefore, the respective capacities of the RTXm-derived RTX1008, RTX908 and RTX770 antigens to form a stable binding structure and tightly engage the CD11b/CD18 receptor on transfected CHO cells was determined. Towards this aim, the capacity of the individual antigens to outcompete the acylated full-length CyaA protein from binding to the CD 11b/CD18 receptor (Masin et al. 2005, Biochemistry 44, pp. 12759-12766) was characterized, using a fluorescently labelled CyaA-AC⁻-Dy647 toxoid and flow cytometry technology (Osicka et al. 2015 Elife 4: e10766). Aliquots of 100,000 CHO cells expressing the CR3 (CD11b/CD18) receptor (Osicka et al, eLife, 2015) were incubated on ice in a final volume of 0.2 ml of cHBSS buffer with fluorescently labelled CyaA-AC⁻-Dy647 toxoid (5.6 nM) in the presence of the tested competitor proteins RTXm, RTX1008, RTX908 and RTX770, respectively, at a range of final concentrations indicated in Figure 6. After 30 minutes of incubation on ice, the Hoechst 33258 vital dye was added to cells to a final concentration of 1 µg/ml and the samples were immediately analyzed for the amount of cell-bound of fluorescently labeled CyaA-AC⁻-Dy647 toxoid by flow cytometry (singlets gating) to determine the mean fluorescence intensity (MFI) of cell-associated CyaA-AC⁻-Dy647. The collected data were analyzed using FlowJo software (Tree Star, Ashland, OR) and the MFI of cell-bound CyaA-AC⁻-Dy647 toxoid in the absence of competitor protein was taken as 100%. As shown in Figure 6, the BlockII-III/V polypeptide antigen (folded in the presence of 2 mM Ca²⁺) was unable to compete with the CyaA-AC⁻-Dy647 toxoid for binding to CD11b/CD18-expressing CHO cells. However, the affinity for the receptor of the RTX-derived antigens was enhanced upon the extension at the N-terminal end to at least the residue 1008, to comprise the entire RTX block I and at C-terminal end to residue 1706, to comprise the sequence of the secretion signal, respectively. The affinity for the receptor and capacity to compete for binding with CyaA-AC⁻-Dy647 toxoid was further enhanced upon N-terminal extension of the RTX-derived antigen to at least the residue 908 by the sequence comprising a portion of the acylated domains with the lysine residue K983 residue. Acylation of this residue by CyaC is essential for tight interaction of the CyaA toxin with the CD11b/CD18 receptor (Masin et al. 2005, Biochemistry 44, pp. 12759-12766) and is crucial for cell penetration capacity and cytotoxic action of the CyaA toxin (Sebo et al. 1990, Gene, 104, pp. 19-24; Hackett et al. Science. 1994, 266, pp. 433-435, Masin et al. 2005, Biochemistry 44, pp. 12759-12766). These data show that the RTX908 and RTX770 antigens bearing the single K983 or both K860 and K983 residues acylated by the co-expressed CyaC enzyme, exhibit higher affinity for the CD11b/CD18 receptor than the RTX1008 antigen devoid of the acylated residues and the BlockII-III/V polypeptide antigen RTXm.

### Example 7. The purified polypeptide antigens RTX770, RTX908 and RTX1008 administered with hydrated aluminium hydroxide adjuvant induce enhanced levels of polyclonal antibodies that block CyaA binding to THP-1 macrophage cells.

The enhanced structural stability of the calcium-loaded extended BlockII-III/V-antigen-derived proteins RTX1008, RTX908 and RTX770, and their enhanced affinity for the CD11b/CD18 receptor binding indicated that these antigens may exhibit more favorable functional folding characteristics once desorbed from the aluminium hydroxide adjuvant of a vaccine injected into the calcium-rich environment of mammalian body. This could translate into a higher capacity to present to the B cells of the immune system the calcium/dependent conformational epitopes of the receptor-binding segment of CyaA and thereby trigger formation of higher levels of anti-CyaA antibodies recognizing the receptor-binding segment of CyaA, thereby neutralizing toxin binding to the CD11b/CD18-expressing myeloid phagocytic cells. Towards testing this hypothesis and to compare the potency of the RTXm and RTX770, RTX908 and RTX1008 antigens in inducing toxin-neutralizing antibodies when administered in a vaccine formulated with hydrated aluminium hydroxide, the purified RTX proteins were formulated with hydrated aluminium hydroxide in PBS and 25 pmols of the individual antigens per dose of 0.5 ml. Groups of 4 Balb/c were immunized twice in two weeks interval intraperitoneally with 0.5 ml of the vaccines containing, respectively, 25 pmols of purified full-length CyaA-AC⁻ toxoid used as standard and positive control (Villarino et al. 2013, Infect. Immun. 81, pp.:2761-2767), or 25 pmol of BlockII-III/V polypeptide antigen RTXm, purified as described under Example 1, or 25 pmol of the antigens RTX1008, RTX908 and RTX770, purified as described in Example 4, respectively. Two weeks after the last injection, mice were euthanized and bled to collect sera. The capacity of the antibodies in the collected sera to block CyaA toxin binding to human THP-1 macrophage cells was determined at serum dilution 1:50 following the procedure described under Example 3. As is shown in Figure 8, by difference to sera from mock-vaccinated mice that did not block CyaA binding to THP-1 cells, preincubation of the toxin with 1:50 diluted serum of mice immunized twice with 25 pmols of the BlockII-III/V polypeptide antigen RTXm caused 35 % inhibition of CyaA binding to THP-1 cells. The 1:50 diluted serum raised against the CyaA-AC⁻ toxoid caused 65 % inhibition of CyaA binding , while the 1:50 diluted sera raised against the extended antigens RTX770, RTX908 and RTX1008, respectively, caused 78%, 75% and 73% inhibition of CyaA binding to THP-1 cells. On an equimolar basis of injected antigen, administered with the hydrated aluminium hydroxide adjuvant and compared to the antigenic capacity of the BlockII-III/V polypeptide antigen RTXm, the extended RTX770, RTX908 and RTX1008 antigens all exhibited a superior capacity to elicit formation of polyclonal antibodies that inhibited, presumably through recognizing and blocking the receptor binding segment of the toxin, the interaction of the CyaA toxin with its CD11b/CD18 receptor on THP-1 macrophage cells. The extended RTX770, RTX908 and RTX1008 thus represent antigens with superior capacity than the BlockII-III/V polypeptide antigen RTXm, in their capacity to undergo calcium-induced folding *in vivo* and present the conformational epitopes of the CR3 receptor-binding segment to B cells, when administered with aluminium adjuvant.

## Claims

1. A polypeptide antigen derived from amino acid sequence of adenylate cyclase toxin protein (CyaA) of *Bordetella pertussis*, comprising a polypeptide sequence consisting of:
(i) amino acid sequence of a fragment of CyaA, having on its N-terminus an amino acid residue in position between 1132-1166 and having on its C-terminus an amino acid residue in position between 1287-1294, and to its C-terminus fused
(ii) amino acid sequence of a fragment of CyaA, having on its N-terminus an amino acid residue in position between 1561-1570 and having on its C-terminus an amino acid residue in position between 1680-1681,
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

2. The polypeptide antigen according to claim 1, comprising a polypeptide sequence consisting of:
(i) amino acid sequence of residues 1166 to 1287, or 1140 to 1290, or 1132 to 1294 of CyaA, or , and to its C-terminus bound
(ii) amino acid sequence of residues 1570 to 1680, or 1570 to 1681, or 1562 to 1680, or 1562 to 1681, or 1565 to 1680, or 1565 to 1681 of CyaA,
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

3. The polypeptide antigen according to claim 1, comprising a polypeptide sequence consisting of:
(i) amino acid sequence of a fragment of the RTX blocks I, II and III of CyaA, having on its N-terminus an amino acid residue in position 984-1008 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681;
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

4. The polypeptide antigen according to claim 1, comprising a polypeptide sequence consisting of:
(i) amino acid sequence of a fragment of the RTX blocks I, II and III of CyaA, having on its N-terminus an amino acid residue in position 905-915 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681;
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

5. The polypeptide antigen according to claim 1, comprising a polypeptide sequence consisting of:
(i) amino acid sequence of a fragment of the RTX blocks I, II and III of CyaA, having on its N-terminus an amino acid residue in position 765-774 and having on its C-terminus an amino acid residue in position between 1287-1294; and to its C-terminus fused
(ii) amino acid sequence of a fragment of the RTX block V of CyaA, having on its N-terminus an amino acid residue in position between 1561-1570 and having on its C-terminus an amino acid residue in position between 1680-1681;
(iii) and optionally fused to the C-terminus of part (ii) an amino acid sequence of the C-terminus of CyaA having on its N-terminus an amino acid residue in position between 1681-1682 and having on its C-terminus an amino acid residue 1706.

6. The polypeptide antigen according to claim 1, comprising a polypeptide sequence selected from SEQ ID NO. 2, SEQ ID NO. 3, SEQ ID NO. 4, SEQ ID NO. 5.

7. A chimeric protein comprising the polypeptide antigen according to any one of the preceding claims fused to at least one heterologous polypeptide, preferably the heterologous polypeptide is fused to N-terminus of said polypeptide antigen.

8. The chimeric protein according to claim 7, wherein the heterologous polypeptide comprises an antigen or an affinity tag and a cleavage site, preferably the chimeric protein has the amino acid sequence SEQ ID NO. 6, SEQ ID NO. 7, SEQ ID NO. 8, SEQ ID NO. 9.

9. A polynucleotide comprising a nucleotide sequence encoding the polypeptide antigen according to any one of claims 1 to 6 or the chimeric protein according to any one of claims 7 to 8; preferably the nucleotide sequence is selected from SEQ ID NO. 10, SEQ ID NO. 11, SEQ ID NO. 12, SEQ ID NO. 13.

10. A vector comprising a polynucleotide according to claim 9.

11. A host cell comprising a polynucleotide according to claim 9, or a vector according to claim 10.

12. A vaccine against a disease caused by a bordetella pathogen, preferably selected from *Bordetella pertussis, Bordetella parapertussis* and *Bordetella bronchiseptica*, in particular against pertussis or whooping cough disease, comprising at least one polypeptide antigen according to any one of claims 1 to 6, and/or at least one chimeric protein according to any one of claims 7 to 8; and at least one pharmaceutically acceptable auxiliary substance, and optionally an adjuvant.

13. The vaccine according to claim 12, wherein the vaccine comprises the polypeptide antigen of any one of claims 1 to 6 or the chimeric protein according to any one of claims 7 to 8, and an aluminium adjuvant; preferably the aluminium adjuvant is aluminium hydroxide or alum.

14. The polypeptide antigen according to any one of claims 1 to 6 or the chimeric protein according to any one of claims 7 to 8 for use in a method of treatment and/or prevention of a disease caused by a bordetella pathogen, preferably selected from *Bordetella pertussis*, *Bordetella parapertussis* and *Bordetella bronchiseptica,* in particular against pertussis or whooping cough disease.

15. A method of treatment and/or prevention of a disease caused by a bordetella pathogen, preferably selected from *Bordetella pertussis*, *Bordetella parapertussis* and *Bordetella bronchiseptica*, in particular against pertussis or whooping cough, comprising the step of administering at least one polypeptide antigen according to any one of claims 1 to 6 or at least one chimeric protein according to any one of claims 7 to 8 to a subject in need of such treatment and/or prevention.
